# EUROPEAN PATENT SPECIFICATION

(11) **EP 1 250 445 B1**
(45) Date of publication and mention of the grant of the patent: **10.10.2007**
(21) Application number: 01946896.6
(22) Date of filing: 29.01.2001
(51) Int. Cl.: C12N 15/82, A01H 1/00, A01H 5/00, C12N 9/10

(54) **ROOT-SPECIFIC EXPRESSION OF TARGET GENES IN PLANTS**
WURZEL-SPEZIFISCHE EXPRESSION VON ZIEL-GENEN IN PFLANZEN
EXPRESSION SPECIFIQUE DES RACINES DE GENES CIBLES DANS DES PLANTES

(30) Priority: 28.01.2000 US 493803
(43) Date of publication of application: 23.10.2002
(73) Proprietor: The Governors of the University of Alberta, Edmonton, Alberta T6G 2E1 (CA)
(72) Inventor: GOOD, Allen, G., Edmonton, Alberta T6H 2X6 (CA)
(74) Representative: Lee, Nicholas John
(86) International application number: PCT/CA2001/000066
(87) International publication number: WO 2001/055433

(56) References cited:
- WO-A-95/09911
- WO-A-97/30163
- JONES JENNIFER T ET AL: "Developmental expression of a turgor-responsive gene that encodes an intrinsic membrane protein." PLANT MOLECULAR BIOLOGY, vol. 28, no. 6, 1995, pages 983-996, XP001016130 ISSN: 0167-4412
- STROEHER V L ET AL: "MOLECULAR CLONING AND EXPRESSION OF A TURGOR-RESPONSIVE GENE IN BRASSICA NAPUS" PLANT MOLECULAR BIOLOGY, NIJHOFF PUBLISHERS, DORDRECHT, NL, vol. 27, 1 February 1995 (1995-02-01), pages 541-551, XP000674453 ISSN: 0167-4412
- GUERRERO F.D. ET AL.: "Turgor-responsive gene transcription and RNA levels increase rapidly when pea shoots are wilted. Sequence and expression of three inducible genes" PLANT MOLECULAR BIOLOGY., vol. 15, 1990, pages 11-26, XP001016037 DORDRECHT., NL ISSN: 0167-4412 cited in the application

## Description

### BACKGROUND OF THE INVENTION

Key factors in the process by which plants grow and thrive are the availability of nutrients to the plant, and the ability of the plant to take up and utilize these nutrients effectively. Plants, in general, have two routes of entry for nutrients: the roots and the leaves. In most plants, elaborate root systems serve to interface with and absorb nutrients from the surrounding soil or water in which the plant grows. To increase the surface area available for contacting and absorbing nutrients, many plants have developed a root structure which includes large numbers ofroot hairs: specially differentiated hairlike root epithelial cells which protrude laterally from the main structure of the root into the surrounding environment. Nutrients which may be readily encountered in many soil and water-based environments and absorbed by the root include, for example, water, nitrogen, phosphorous, magnesium, potassium, and sulfur.

One nutrient which is only inefficiently obtained through the roots, however, is carbon dioxide. Plants fix carbon dioxide into glucose through photosynthesis; uptake of carbon dioxide is therefore critically important to the continued growth and survival of the plant. The bulk of carbon dioxide uptake in most plants occurs through the leaves from the surrounding air. A few plants (*e.g.,* orchids) forego root-based uptake of nutrients in favor of a solely leaf-based uptake system (in which, for example, nutrients dissolved in water are taken up by the leaves), but the bulk ofplants utilize both root and leaf-based nutrient absorption systems.

Many nutrients taken up by plants require processing prior to becoming usable in plant metabolic pathways. Nitrogen is one such nutrient, and is also, along with phosphorous and potassium, one of the three nutrients which primarily limit plant growth. Nitrogen sources are often the major components in fertilizers (Hageman and Lambert, 1988, In: Corn and Corn Improvement, 3rd ed., Sprague & Dudley, American Society of Agronomy, pp. 431-461). Since nitrogen is usually the rate-limiting element in plant growth, most field crops have a fundamental dependence on inorganic nitrogenous fertilizer. The nitrogen source in fertilizer is usually ammonium nitrate, potassium nitrate, or urea. A significant percentage of the costs associated with crop production results from necessary fertilizer applications. However, it is known that most of the nitrogen applied is rapidly depleted by soil microorganisms, leaching, and other factors, rather than being taken up by the plants.

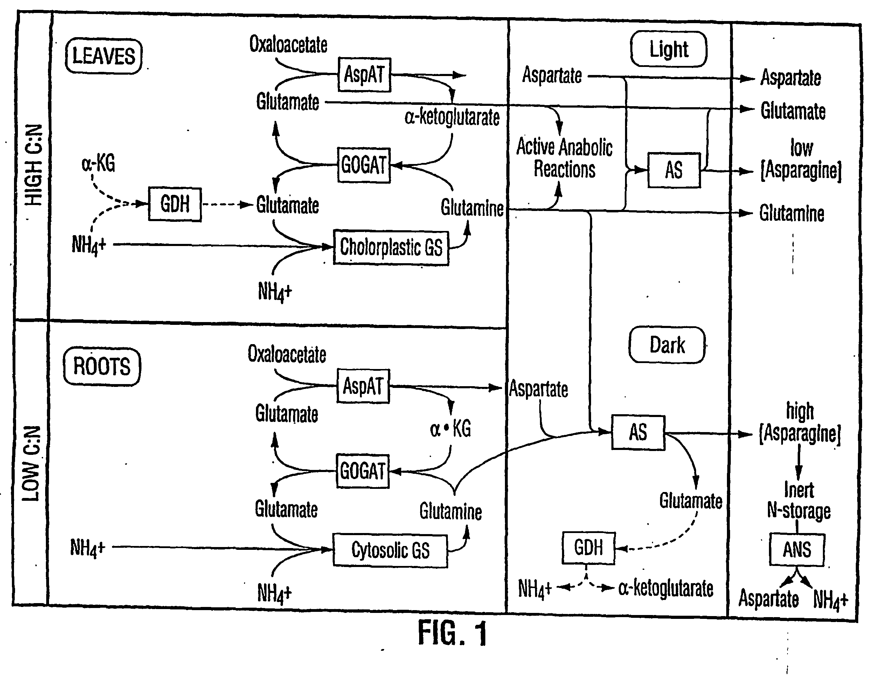

Nitrogen is taken up by plants primarily as either nitrate (NO₃⁻) or ammonium (NH₄⁺). Some plants are able to utilize the atmospheric N₂ pool through a symbiotic association with N₂-fixing bacteria or ascomycetes. In well aerated, non-acidic soils, plants take up NO₃⁻which is converted to NH₄⁺. In acidic soils, NH₄⁺ is the predominate form of inorganic nitrogen present and can be taken up directly by plants. NH₄⁺ is then converted to glutamine and glutamate by the enzymes glutamine synthetase (GS) and glutamate synthase (GOGAT). The glutamine and glutamate can be converted into a variety of amino acids, as shown in Figure 1.

Although some nitrate and ammonia can be detected in the transporting vessels (xylem and phloem), the majority of nitrogen is first assimilated into organic form (e.g., amino acids) which are then transported within the plant. Glutamine, asparagine and aspartate appear to be important in determining a plant's ability to take up nitrogen, since they represent the major long-distance nitrogen transport compounds in plants and are abundant in phloem sap. Aside from their common roles as nitrogen carriers, these amino acids have somewhat different roles in plant nitrogen metabolism. Glutamine is more metabolically active and can directly donate its amide nitrogen to a large number of substrates. Because of this reactivity, glutamine is generally not used by plants to store nitrogen. By contrast, asparagine is a more efficient compound for nitrogen transport and storage compared to glutamine because of its higher N:C ratio. Furthermore, asparagine is also more stable than glutamine and can accumulate to higher levels in vacuoles. Indeed, in plants that have high nitrogen assimilatory capacities, asparagine appears to play a dominant role in the transport and metabolism of nitrogen (Lam et al, 1995, Plant Cell 7: 887-898). Because of its relative stability, asparagine does not directly participate in nitrogen metabolism, but must be first hydrolysed by the enzyme asparaginase (ANS) to produce aspartate and ammonia which then can be utilized in the synthesis of amino acids and proteins.

However, in addition to aspartate and asparagine, a number of other amino acids can act as storage compounds. The total amount of free amino acids has been shown to change with specific stresses, both biotic and abiotic, different fertilizer regimes and other factors (Bohnert et al., 1995, Plant Cell 7:1099-1111). For example, during drought stress many plants maintain their turgor by osmotic adjustment (Turner, 1979, Stress Physiology in Crop Plants, pp. 181-194). Osmotic adjustment, *i.e.* a net increase in solutes leading to a lowering of osmotic potential, is one of the main mechanisms whereby crops can adapt to limited water availability (Turner, ibid; Morgan, 1984, Annu. Rev. PlantPhysiol. 35: 299-319). The solutes that accumulate during osmotic adjustment include sugars, organic acids and amino acids, such as alanine, aspartate and proline and glycine betaine (Good and Zaplachinski, 1994, Physiol. Plant 90: 9-14; Hanson and Hitz, 1982, Annu. Rev. Plant Physiol. 33: 163-203; Jones and Turner, 1978, Plant Physiol. 61: 122-126). Corn, cotton, soybean and wheat have all demonstrated osmotic adjustment during drought (Morgan, *ibid.).* One of the best characterized osmoregulatory responses is the accumulation of proline (Hanson and Hitz, *ibid.).* In some tissues, proline levels increase as much as 100-fold in response to osmotic stress (Voetberg and Sharp, 1991, Plant Physiol. 96: 1125-1130). The accumulation of proline results from an increased flux of glutamate to pyrroline-5-carboxylate and proline in the proline biosynthetic pathway, as well as decreased rates of proline catabolism (Rhodes et al., 1986, Plant Physiol. 82:890-903; Stewart et al., 1977, Plant Physiol. 59:930-932). The concentrations of alanine and aspartate have been shown to increase 3.6 and 4.1-fold, respectively, during drought stress in *Brassica napus* leaves, whereas glutamate levels increased 5.5-fold (Good and Maclagan, 1993, Can. J. Plant Sci. 73: 525-529). Alanine levels declined after rewatering of the plants whereas aspartate levels remained high. Pyruvate levels showed a similar pattern, increasing 2.2-fold after 4 days of drought, followed by the return to control levels upon rehydration. However, 2oxoglutarate levels remained relatively constant during drought stress and rehydration. One of the factors that may determine the value of a specific amino acid as an osmoprotectant may be its use as a carbon or nitrogen storage compound.

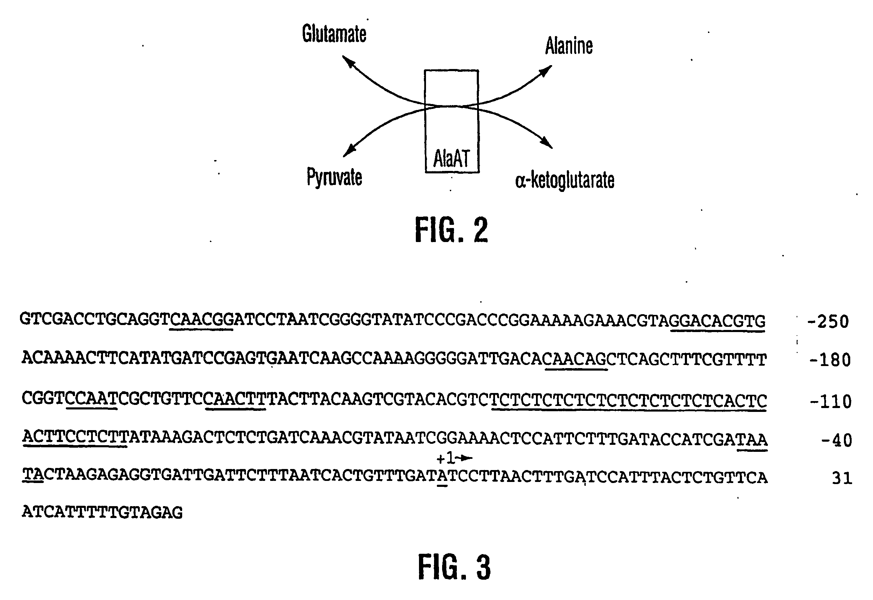

Alanine is one of the more common amino acids in plants. In *Brassica* leaves under normal conditions, alanine and aspartate concentrations are roughly equal and have been found to be twice that of asparagine concentrations. In comparison, glutamate levels were double that of alanine or aspartate (Good and Zaplachinski, *ibid.*). Alanine is synthesized by the enzyme alanine aminotransferase (AlaAT) from pyruvate and glutamate in a reversible reaction (Goodwin and Mercer, 1983, Introduction to Plant Biochemistry 2nd Ed., Pergamon Press, New York, N.Y., pp. 341-343), as shown in Figure 2. In addition to drought, alanine is an amino acid that is known to increase under other specific environmental conditions such as anaerobic stress (Muench and Good, 1994, Plant Mol. Biol. 24:417-427; Vanlerberge et al.,1993, Plant Physiol. 95:655-658). Alanine levels are known to increase substantially in root tissue under anaerobic stress. As an example, in barley roots alanine levels increase 20 fold after 24 hours of anaerobic stress. The alanine aminotransferase gene has also been shown to be induced by light in broom millet and when plants are recovering from nitrogen stress (Son et al., 1992, Arch. Biochem. Biophys. 289: 262-266). Vanlerberge *et al.* (1993) have shown that in nitrogen starved anaerobic algae, the addition of nitrogen in the form of ammonia resulted in 93% of an N₁₅ label being incorporated directly into alanine. Thus, alanine appears to be an important amino acid in stress response in plants.

The nitrate transporter genes, nitrate reductase (NR) and nitrite reductase (NiR) (Crawford, 1995, Plant Cell 7:859-868; Cheng et al, 1988, EMBO J. 7:3309-3314) have been cloned and studied, as have many of the genes encoding enzymes involved in plant nitrogen assimilation and metabolism. Glutamine synthetase (GS) and glutamate synthetase (GOGAT) have been cloned (Lam et al., ibid.; Zehnacker et al., 1992, Planta 187:266-274; Peterman and Goodman, 1991, Mol. Gen. Genet. 230:145-154) as have asparaginase (ANS) and aspartate aminotransferase (AspAT) (Lam et al., ibid; Udvardi and Kahn, 1991, Mol. Gen. Genet. 231:97-105). An asparagine synthetase (AS) gene has been cloned from pea (Tsai and Coruzzi, 1990, EMBO J. 9:323-332). Glutamate dehydrogenase has been cloned from maize (Sakakibara et al., 1995, Plant Cell Physiol. 36(5):789-797. Alanine aminotransferase has been cloned by Sonet al. (1993, Plant Mol. Biol. 20:705-713) and by Muench and Good, (1994, Plant Mol. Biol. 24:417-427). Among the plant nitrogen assimilation and utilization genes, the most extensively studied are the glutamine synthetase and asparagine synthetase genes.

In plants, genetic engineering of nitrogen assimilation processes has yielded varied results. Numerous studies examining constitutive overexpression of glutamine synthetase (GS) have failed to report any positive effect of its overexpression on plant growth. These studies include, for example: Eckes et al. (1989, Molec. Gen. Genet. 217: 263-268) using transgenic tobacco plants overexpressing alfalfa GS; Hemon et al.(1990, Plant Mol. Biol. 15: 895-904) using transgenic tobacco plants overexpressing bean GS in the cytoplasm or mitochondria; and Hirel et al. (1992, Plant Mol. Biol. 20: 207-218) using transgenic tobacco plants overexpressing soybean GS. One study, by Temple et al. (1993, Mol. Gen. Genet. 236: 315-325), has reported increases in total soluble protein content in transgenic tobacco plants overexpressing an alfalfa GS gene and similar increases in total soluble protein content in transgenic tobacco plants expressing antisense RNA to a GS gene. WO 97/30163 reports an increased biomass seen in plants transformed with the alanine amino transferase gene under control of the brassica turgor gene-26 promoter and grown under low nitrogen conditions, compared to controls.

There has been a report that plants engineered to constitutively overexpress an alfalfa GS gene grow more rapidly than control, wild-type plants (Eckes et al., 1988, Australian published patent application no. 17321/88). Another report (Coruzzi and Brears 1994, WO 95/09911) introduced GS, GOGAT and AS constructs under the control of a constitutive Cauliflower Mosaic Virus 35S (CaMV35S) promoter. This document showed that the transgenic plants had increased fresh weight and growth advantage over controls. Thus, there appears to be no clear direction on the effect of constitutive overexpression of nitrogen assimilation enzymes on plant growth.

Like nitrogen, water represents another critically important plant nutrient. Maintenance of normal growth and function in plants is dependent on a relatively high intracellular water content. Drought, low temperature and high salinity are all environmental stresses that alter cellular water balance and significantly limit plant growth and crop yield (Morgan, ibid.). Many physiological processes change in response to conditions that reduce cellular water potential, including photosynthesis, stomatal opening and leaf, stem and root growth (Hanson and Hitz, *ibid*.) Along with physiological responses, metabolic changes can also occur during water loss. One of the most notable changes is in the synthesis and accumulation of low molecular weight, osmotically active compounds, as noted above.

Changes in gene expression also occur during osmotic stress. A number of genes have recently been described that are induced by drought (reviewed by Skiver and Mundy, 1990, Plant Cell 2:503-512).

It is an object of the invention to overcome disadvantages of the prior art.

The above obj ect is met by the combinations of features of the main claim, the sub-claims disclose further advantageous embodiments of the invention.

### SUMMARY OF THE INVENTION

The present invention provides a method for directing root-specific expression of a target gene, comprising: producing a plant from a transformed plant cell such that expression of a target gene occurs preferentially in the root, wherein the transformed plant cell comprises a target gene in operative linkage with a brassica turgor gene 26 promoter element as defined in SEQ ID NO: 1, or a fragment thereof which fragment directs expression of the target gene preferentially in the root.

The expression of the target gene may be further environmentally or developmentally regulated. The target gene may encode an enzyme involved in nitrogen assimilation and/or metabolism. For example, the enzyme may be alanine dehydrogenase, glutamine synthetase, asparagine synthetase, glutamate synthase, asparaginase, alanine aminotransferase, aspartate aminotransferase or glutamate dehydrogenase.

The method of the invention may be carried out in a plant such as, canola, barley, corn, rice, tobacco, soybean, cotton, alfalfa, tomato, wheat or potato.

The fragment of a brassica turgor gene 26 promoter element as defined in SEQ ID NO: 1 which directs expression of the target gene preferentially in the root may be, for example, the fragment defined in any one of SEQ ID NO's: 2, 3, 4, 5 or 6.

Plants face a wide variety of nonoptimal environmental conditions during growth and development. Such conditions may include water limitation, excess salinity, alkaline or acidic soil, infestation by pests, disease, or temperature stress, any of which individually may significantly hinder crop growth and/or yields. The present invention provides novel methods by which plants, and seeds thereof, may be engineered to grow and thrive under environmental conditions usually not conducive to the development of the plant. The invention provides methods by which one or more selected genes (e.g., genes whose products may improve the ability of the plant to grow and thrive under one or more environmentally adverse conditions) may be expressed in the root. It is further possible, according to the methods of the invention, to express the selected gene or genes in the root in an inducible fashion, such that the expression of the gene product may take place only under desired environmental, temporal, or developmental conditions, or in response to a specific event (e. g., injury by a pest). Using the methods of the invention, plants may be improved for growth and development under environmental conditions usually unsuitable for growth of the plant. Furthermore, the methods of the invention permit the genetic engineering of a plant to alter one or more plant characteristics in only selected tissues of the plant.

Expression of the target gene may be further environmentally or developmentally-regulated. The environmental or developmentally-regulated expression may take place under conditions of osmotic stress.

In a preferred embodiment of the present invention, the target gene is selected from the group including: genes encoding proteins which alter nutrient content, genes encoding proteins involved in phytoremediation, genes encoding proteins conferring pesticide resistance, genes encoding structural proteins, genes producing pharmaceutical proteins or enzymes which produce pharmaceutical compounds, genes encoding proteins involved in nutrient uptake or utilization, and genes encoding proteins involved in plant growth. In another preferred embodiment, expression of the target gene is also environmentally or developmentally regulated.

Also disclosed herein is a seed containing a gene in operative linkage with a genetic regulatory element which directs the root-specific expression of the target gene. The target gene may be selected from the group consisting of: genes encoding proteins which alter nutrient content, genes encoding proteins involved in phytoremediation, genes encoding proteins conferring pesticide resistance, genes encoding structural proteins, genes producing pharmaceutical proteins or enzymes which produce pharmaceutical compounds, genes encoding proteins involved in nutrient uptake or utilization, and genes encoding proteins involved in plant growth. Expression of the target gene may be further environmentally or developmentally regulated.

A method for increasing biomass of a plant growing under one or more environmentally adverse conditions may comprise:
transforming a plant with a target gene in operative linkage with a btg-26 promoter element to produce a transformed plant, the target gene encoding an enzyme involved in nitrogen assimilation or metabolism; and
growing the transformed plant. Preferably the enzyme is AlaAT.

A method for increasing biomass of a plant growing under conditions of low nitrogen may comprise;
transforming a plant with a target gene in operative linkage with a btg-26 promoter element to produce a transformed plant, the target gene encoding an enzyme involved in nitrogen assimilation; and
growing the transformed plant. Preferably, the enzyme is AlaAT.

A method for increasing the biomass of a plant growing under one or more environmentally adverse conditions may comprise;
transforming a plant with gene encoding AlaAT in operative linkage with a promoter element to produce a transformed plant; and
growing the transformed plant. Preferably the promoter is btg-26.

A method for increasing seed yield of a plant may comprise;
transforming the plant with a target gene in operative linkage with a btg-26 promoter element, to produce a transformed plant, the target gene encoding an enzyme involved in nitrogen assimilation or metabolism; and
growing the transformed plant. Preferably the enzyme is AlaAT.

### BRIEF DESCRIPTION OF THE DRAWINGS

These and other features of the invention will become more apparent from the following description in which reference is made to the appended drawings wherein:
- **Figure 1**: shows major pathways of nitrogen assimilation and metabolism in plants. (Adapted from Lam et al., 1995, Plant Cell 7:889 where Arabidopsis is used as a model system). Some of the enzymes of the nitrogen assimilation and amide amino acid metabolism pathways are shown. Different isoenzymes are known for some of these enzymes which may play different roles under different environmental and tissue conditions. Nitrogen assimilation occurs primarily through the activities of glutamine synthetase (GS) and glutamate synthase (GOGAT). While not indicated as such, aspartate aminotransferase also catalyses the reverse reaction. The roles of glutamate dehydrogenase (GDH) are postulated, as indicated by the dashed lines.
- **Figure 2**: shows pathway for alanine biosynthesis by the enzyme alanine aminotransferase (AlaAT) (From Goodwin and Mercer, 1983).
- **Figure 3**: shows the DNA sequence of the Brassica napus btg-26 promoter.

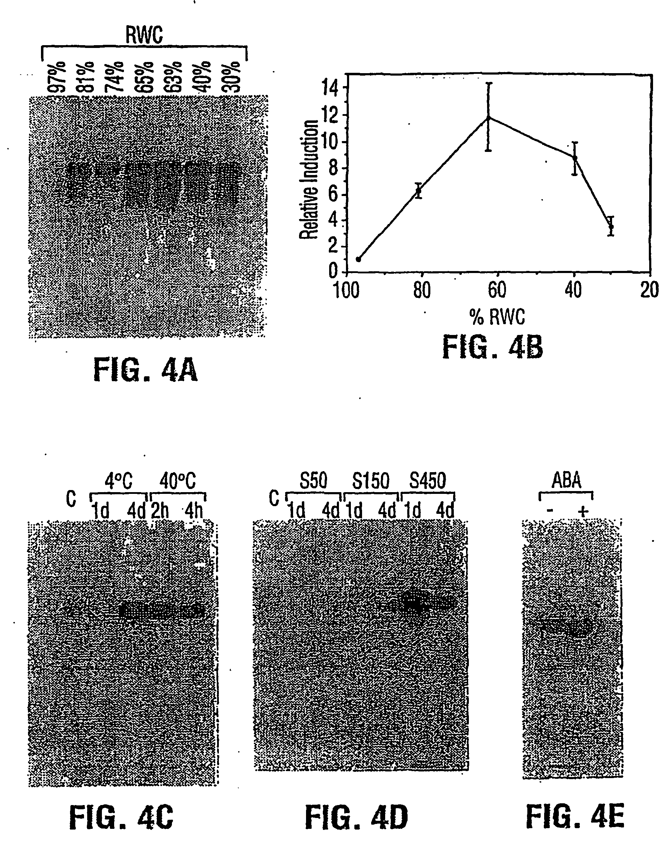

- **Figure 4**: shows Northern quantitative analysis of the expression of btg-26. Figure 4A shows Northern blot analysis of btg-26 expression during droughting. Total RNA (10 mg) from leaf tissue taken from control plants having 97% relative water content (97% RWC) and plants dehydrated to the % RWC's as, indicated, was fractionated on a 1.2% agarose formaldehyde gel andprobedwithbtg-26 genomic DNA. Figure 4B shows quantitative analysis of btg-26 induction. Each time point represents the mean induction determined from three independent slot blots and two Northern blots. All blots were reprobed with a cyclophilin cDNA control to correct for loading error. Induction is determined relative to the level of expression in fully hydrated plants (97%). Figure 4C shows Northern blot analysis of btg-26 expression during cold acclimation and heat shock. Total RNA (10 mg) from leaf tissue taken from control plants (C) or plants exposed to 4°C for one or four days or exposed to 40°C for two or four hours. The RNA was fractionated on a 1.2% agarose formaldehyde gel and probed with btg-26 genomic DNA. **Figure 4D** shows Northern blot analysis of btg-26 expression during salinity stress. Total RNA (10 mg) from leaf tissue taken from control plants (C) or plants exposed to salinity stress by watering with 50mM NaCl (S50), 150mM NaCl (S150) or 450mM NaCl (S450) for one or four days. The RNA was fractionated on a 1.2% agarose formaldehyde gel and probed with btg-26 genomic DNA. **Figure 4E** shows Northern blot analysis of btg-26 expression during exposure to abscisic acid (ABA). Total RNA (10 mg) from leaf tissue taken from plants soaked for one day in a solution containing either 0 iM (-) or 100 iM ABA (+). The RNA was fractionated on a 1.2% agarose formaldehyde gel and probed with btg-26 genomic DNA.
- **Figure 5**: shows nucleotide and deduced amino acid sequence of the AlaAT cDNA from barley.
- **Figure 6**: shows the plasmid construct p25.
- **Figure 7**: shows constructs containing the AlaAT coding region and the CaMV, btg-26 and trg-31 promoters that were used for the transformation of *Brassica napus* plants. Figure 7A shows pCa2/AlaAT/NOS; Figure 7B shows pbtg-26/AlaAT/NOS; and Figure 7C shows ptrg-31/AlaAT/NOS.
- **Figure 8**: shows plasmid construct pCGN1547 used in producing the overexpressed/AlaAT or drought inducible/AlaAT transformants.
- **Figure 9**: shows *Brassica napus* plants grown under nitrogen starved conditions for three weeks followed by drought for 3 days. The plants are identified as A, B and C, as follows: Plant A is a control, wild-type plant; Plant B contains a CaMV/AlaAT construct; and Plant C contains a btg-26/AlaAT construct.
- **Figure 10**: shows GUS *in vivo* staining of btg-26/GUS transgenic plants. Panel A shows wild-type plants on the left and transgenic plants on the right. Panel B shows the root tips of stained transgenic plants. Panel C shows a cross section of the absorption zone of the root tips of the transgenic plants. Panel D shows a cross section of the division zone of root tips of the transgenic plants.
- **Figure 11**: shows GUS activity in shoots versus GUS activity in roots of btg-26/GUS transgenic plants. This figure shows a bar graph of the amount of GUS activity present in extracts of root and shoot from plants expressing the btg-26/GUS transgene, and the root:shoot ratio of GUS activity in these plants.
- **Figure 12**: shows Southern blot analysis of RT-PCR reactions amplifying AlaAT from leaf (L) and root (R) total RNA. Relative densitometric analysis of the 381 bp product of the RT-PCR reaction is indicated below each lane.
- **Figure 13**: shows transgenic (btg-26/AlaAT) and control (wild-type) leaf AlaAT activity under low and high nitrogen conditions.
- **Figure 14**: shows AlaAT activity in shoots of wild-type, cv. Westar, and transgenic btg-26/AlaAT line 8 1 B plants grown hydroponically with 0.5 mM nitrate after 36 hours of salt treatment.
- **Figure 15**: shows AlaAT activity in roots of wild-type, cv. Westar, and transgenic btg-26/AlaAT line 81B plants grown hydroponically with 0.5 mM nitrate after 36 hours of salt treatment.
- **Figure 16**: shows the effect of salinity on biomass accumulation of wild type, cv. Westar, and transgenic, btg-26/AlaAT, line 81B plants. Panel A shows wild-type shoots, panel B shows btg-26/AlaAT shoots, panel C shows wild-type roots and panel D shows btg-26/AlaAT roots.
- **Figure 17**: shows the effect of salinity on the growth of wild-type, cv. Westar, and transgenic, btg-26/AlaAT, line 81B plants. Wild-type plants are on the left side of the picture, and transgenic plants are on the right side of the picture.
- **Figure 18**: shows immunolocalization of expressed AlaAT protein in wild type and transgenic (btg-26/AlaAT) plants. Panel A shows control wild-type untreated root, panel B shows transgenic untreated root, panel C shows wild-type root treated with 150 mM NaCl, and panel D shows transgenic root treated with 150 mM NaCl.
- **Figure 19**: shows the increase in plant biomass in transgenic plants expressing btg-26/AlaAT under field conditions and treated with fertilizer containing low or high levels of nitrogen. Figure 19A shows the dry weight obtained from transgenic plants expressing btg-26/Ala/AT (53F and 81B) and control plants (Westar). Figure 19B shows seed yields (g/plot harvested) obtained from transgenic plants expressing btg-26/Ala/AT (53F and 81B) and control plants (Westar).

### DESCRIPTION OF PREFERRED EMBODIMENT

The following description is of a preferred embodiment by way of example only and without limitation to the combination of features necessary for carrying the invention into effect.

### I. Definitions

The language "tissue-specific expression of a target gene" is known in the art and includes the expression of a target gene in only selected tissues; although the target gene may be present in multiple tissues, it is expressed in only a subset of those tissues. Such selective expression may be due to the influence of one or more regulatory genetic elements, e.g., promoter, repressor, or enhancer elements.

The language "target gene" is art-recognized, and includes any gene which is desirably expressed in one or more selected plant tissues. Examples of target genes which may advantageously be utilized in conjunction with the methods of the invention include nitrogen assimilation and/or utilization genes, stress resistance genes, disease and pest resistance genes, and genes related to nutrient uptake and utilization.

The language "plant" is art-recognized, and includes any monocotyledenous or dicotyledenous plant. Preferred plants for use in the invention include canola, barley, corn, rice, tobacco, soybean, cotton, alfalfa, tomato, wheat, potato, and certain tree genera, including conifers and *Populus* species.

The language "operative linkage" is art-recognized, and includes the placement of a target gene relative to a nucleic acid regulatory sequence such that the expression of the target gene is controlled by the regulatory sequence. This regulatory sequence can have a positive effect on the expression of the target gene (e.g., the regulatory sequence is a promoter or an enhancer element), or the regulatory sequence can have a negative effect on the expression of the target gene (e. g., the regulatory sequence is a repressor element). The regulatory sequence may be physically located 5' or 3' of the target gene, may be within the coding sequence of the target gene, or may be contained on an intron within the target gene.

The language "phytoremediation" is art-recognized, and includes the utilization of one or more plants for the purpose of extracting compounds from the soil or water which are harmful to humans or animals and either a) storing these compounds in the mass of the plant for more convenient disposal, or, preferably, b) converting these compounds within the plant to less harmful substances.

### Methods of the Invention

The present invention is drawn to the production of plants which express one or more target genes in a root-specific fashion. Seeds are described containing one or more target genes under the control of a promoter element which specifically directs the root-specific expression of the gene. By the methods of the invention, it is possible to produce plants having one or more desired traits or properties in selected tissues; e.g., to alter specifically the genetic and/or physiological properties of the roots of the plant. The invention provides methods of producing plants having root-specific expression of one or more desired genes, using the brassica turgor protein-26 promoter element.

In one embodiment, the invention provides a method for directing the root-specific expression of one or more target genes in a plant, in which a plant is produced from a plant cell containing the target gene in operative linkage with a genetic regulatory element that directs the root-specific expression of the target gene(s), such that the plant has expression of the target gene within a root. The genetic regulatory element is a brassica turgor gene-26 promoter element.

In another embodiment, the invention provides a method for directing the root-specific and environmental or developmental-specific expression of one or more genes in a plant, in which a plant is produced from a plant cell containing the target gene in operative linkage with a genetic regulatory element that directs the root-specific and environmental or developmental-specific expression of the target gene(s), such that the plant has expression of the target gene only under the selected environmental or developmental conditions and within the root of the plant. The genetic regulatory element is a brassica turgor gene-26 promoter element, and the desired environmental condition is preferably drought or saline stress.

Seeds containing constructs in which a promoter directs both the tissue-specific and optionally environmental or developmental specific expression of one or more target genes are disclosed herein.

The methods of the invention are further explicated below.

### Genetic Constructs of the Invention

### A. Genetic Regulatory Elements

The methods for the production of plants having tissue-specific expression of one or more target genes are accomplished through the use of a genetic regulatory element which directs the tissue-specific expression of the target gene(s). Regulatory elements may be either negative or positive in activity: a plant tissue-specific promoter or enhancer element permits the expression of the target gene(s) in one or more specific tissues, whereas a plant tissue-specific repressor suppresses the expression of the target genes in one or more specific tissues, while expression in the other tissue(s) continues unabated. For the purposes of the invention, it will be understood that promoter sequences constitute the genetic regulatory elements of the invention.

Tissue-specific promoters can be either homologous or heterologous to the plant to which they are to be used. For example, promoters which direct tissue-specific expression of a gene in plants but which are derived from, e.g., viruses, bacteria, insects, or animals may be used. Promoters which direct the expression of an operatively linked gene in one or more plant tissues while excluding expression of the linked gene in one or more other plant tissues may be used. Appropriate plant tissues include but are not limited to e. g.; root, leaf, petal, sepal, stamen, anther, stigma, ovary, style, pistil, epidermis, phloem, xylem, cortex, pith, cambium, stem or trunk, root hair, petiole, fruit and tuber.

It will be understood by one skilled in the art that modifications may be made to the promoters useful in the methods of the invention to improve or modulate the activity of the promoter. Multiple copies of a selected promoter may be operatively linked to a single target gene to thereby alter the expression level of the linked gene, or a selected promoter may be operatively linked to one or more target genes such that the expression of each target gene is coordinately regulated. A promoter may be of any size appropriate to permit the tissue-specific functioning of the promoter. A promoter may be modified (e. g., by mutagenesis, deletion, insertion, or truncation) to alter the degree to which the operatively linked gene is expressed in the selected tissue, or to alter the specificity of tissue expression directed by the promoter. Further, the placement of the promoter relative to the operatively linked target gene may be modulated (e. g., moved further away or closer together) to attain a desired level of promoter-directed expression.

In one embodiment, the promoter not only directs the expression of an operatively linked gene in one or more selected tissues of the plant, but also directs expression of the gene in response to a specific environmental orphysiological condition. For example, the promoter may be activated under conditions of stress (e.g., drought stress, saline stress, temperature stress, oxygen stress, pH stress, or heavy metal stress), under conditions of nutrient deprivation, under conditions of attack by disease or pests, or under specific developmental conditions (e.g., upon sprouting, fruiting, or seed production). Through the use of such a promoter it is possible to activate the expression of a target gene in both a tissue-specific and a condition-specific manner.

The promoter for use in methods of the invention is the brassica turgor gene 26 (btg-26) promoter, the sequence of which is set forth as SEQ ID NO:1, and expression of the operatively linked gene is to the root of the transgenic plant under conditions of osmotic stress. One or more fragments (e. g., CAACGG (SEQ ID NO:2), GGACACGTGAC (SEQ ID NO:3), CAACAG (SEQ ID NO:4), CAACTT (SEQ ID NO:5), and TCTCTCTCTCTCTCTCTCTCTCTCACTCACTTCCTCTT (SEQ ID NO: 6)) of the btg-26 promoter can be operatively linked to the target gene and,direct specific expression of the target gene.

### B. Target Genes

A target gene of the invention may be any gene which is desirably expressed in a tissue-specific manner in a plant. General classes of target genes which may be advantageously employed in the methods and constructs of the invention include genes encoding plant structural proteins, genes encoding proteins involved in the transport and/or uptake of nutrients, genes encoding enzymes and proteins involved in nutrient utilization, genes encoding proteins involved in plant resistance to pesticides or herbicides, genes encoding proteins involved in plant resistance to nematodes, viruses, insects, or bacteria, genes encoding proteins involved in plant resistance to stress (e.g., osmotic, temperature, pH, or oxygen stress), genes encoding proteins involved in stimulation or continuation of plant growth, genes encoding proteins involved in phytoremediation, or genes encoding proteins having pharmaceutical properties or encoding enzymes which produce compounds having pharmaceutical properties. Further, the target gene may not be a gene at all, but rather a genetic sequence which, when transcribed, is antisense to a native sequence, the transcription and translation of which is desired to be suppressed.

In one embodiment, the genes of interest are those encoding enzymes in the assimilation and/or metabolism of nitrogen and, in particular, those which assimilate ammonia into amino acids or use the formed amino acids in biosynthetic reactions. These enzymes include, but are not limited to, alanine dehydrogenase, glutamine synthetase (GS), asparagine synthetase (AS), glutamate synthase (also known as glutamate 2:oxogluturate amino transferase and GOGAT), asparaginase (ANS), glutamate dehydrogenase (GDH), aspartate aminotransferase (AspAT) (activities are shown in Figure 1) and alanine aminotransferase (AlaAT) (activity shown in Figure 2).

The target gene may be a gene naturally expressed in the selected plant, or it may be heterologous to the selected plant. The gene may originate from any source, including viral, bacterial, plant or animal sources. Preferably, the gene is heterologous to the promoter to which it is linked, in that it is not linked to an unmodified, inducible promoter that the gene is naturally linked to.

The target gene can be modified in any suitable way in order to engineer a gene or plant with desirable properties. In one embodiment, the gene is modified to be transcribable and translatable in a plant system; for example, the gene can be modified-such that it contains all of the necessary poly-adenylation sequences, start sites and termination sites which allow the coding sequence to be transcribed to mRNA (messenger ribonucleic acid) and the mRNA to be translated in the selected plant system. Further, the target gene may be modified such that its codon usage is more similar to that of native genes of the selected plant. Such target gene modifications and the methods by which they may be made are well known in the art.

### Vectors

Tissue-specific promoter-target gene constructs are most efficiently introduced into a plant cell or plant protoplast through the use of a vector. Examples of cloning or expression vectors suitable for use with the invention are plasmids, cosmids, viral DNA or RNA, and minichromosomes. Appropriate plant vectors are well known in the art (see, e.g., Clark, M., ed. (1997) Plant Molecular Biology: A Laboratory Manual. Springer Verlag, ISBN:3540584056).

Vectors can advantageously contain one or more bacterial or plant-expressible selectable or screenable markers, such that incorporation of the vector into a plant cell or protoplast can be monitored. It is preferable that such selectable or screenable markers confer a readily detectable phenotype, such as resistance to an otherwise toxic compound (e.g., kanamycin resistance), or a colorimetric or luminescent reaction upon incubation of the plant with an appropriate substrate (e.g., a-glucuronidase (GUS) or luciferase genes). Such marker genes are well known in the art.

Either the tissue-specific promoter or the target gene may be incorporated into a vector and introduced into a selected plant. 5' and 3' flanking sequences homologous to the up-and downstream genetic sequences of the location in the plant genome where recombination of the introduced promoter sequence or gene is desired may surround the promoter or target gene in the vector such that homologous recombination takes place upon introduction of the vector into the plant cell or protoplast. Through such homologous recombination, plants may be produced having a heterologous target gene under control of a native tissue-specific promoter, or a heterologous tissue-specific promoter in operative linkage to a native target gene.

### D. Transformation of Plant Cells

The gene construct can be introduced to a plant cell by any useful method. A large number of processes are available and are well known to deliver genes to plant cells. One of the best known methods involves the use of *Agrobacterium* or similar soil bacteria as a vector. Target tissues are co-cultivated with

*Agrobacterium,* which inserts the gene of interest into the plant genome, as is described by U.S. Patent 4,940,838 (Schilperoort et al.), and Horsch et al. 1985, Science 227:1229-1231. Alternative gene transfer and transformation methods useful in the present invention include, but are not limited to liposomes, electroporation or chemical-mediated uptake of free DNA, calcium phosphate co-precipitation techniques, targeted microprojectiles and micro-or macroinjection. Such transformation methods are well documented in the art and are disclosed, for example, in Ausubel et al., Current Protocols in Molecular Biology, Wiley: New York, and references contained therein. It will be understood by one skilled in the art that the method chosen for plant cell or protoplast transformation will in large part be determined by the nature of the tissue-specific promoter-target gene construct and/or the vector containing it.

### Plants

The methods and genetic constructs disclosed herein may be used to produce a plant of any species capable of utilizing the promoter such that the transgenic plant has tissue-specific expression of one or more desired genes. Both monocotyledenous and dicotyledenous plants are amenable to such alteration. The invention is intended to be particularly applicable to, for example, crop plants (especially those of the genus *Brassica),* ornamental plants, and trees (particularly conifers and the genus *Populus).* Particularly suitable plants for the practice of the present invention include, but are not limited to, canola, barley, corn, rice, tobacco, soybean, cotton, alfalfa, tomato, wheat, potato, aspen, cottonwood, conifers and poplar.

The transgenic plants and seeds produced according to the present invention may be further useful in breeding programs for the production of plant species having more than one desired trait (e.g., two transgenic plants of the invention each having expression of a desired transgene in differing plant tissues may be crossed to result in progeny transgenic plants having tissue-specific expression of both transgenes; or two transgenic plants of the invention each having expression of a different desired transgene in the same plant tissue may be crossed to result in progeny transgenic plants having tissue-specific expression of both transgenes). In this fashion it is possible to produce transgenic plants having a combination of desirable traits in selected tissue(s) of the plant.

Furthermore, it will be understood by one skilled in the art that different species of plants may be more or less amenable to genetic manipulation in general, and that, therefore, it may be advantageous to first transform a related species of the desired plant by the methods of the invention and with the constructs described herein and to subsequently introduce the tissue-specific expression of the target gene into the desired plant species by crossbreeding techniques. Such techniques and appropriately related plant species are well known to one skilled in the art.

Plant cells or protoplasts that have been transformed with the gene construct described herein can be regenerated into differentiated plants using standard nutrient media supplemented with shoot-inducing or root-inducing hormone, using methods known to those skilled in the art (see, for example, Shahin, E. A. U. S. Patent 4,634,674 and references therein). Seeds may additionally be harvested from such transgenic plants using methods well known in the art and further used to regrow transgenic plants and hybrids.

### Uses of the Invention

The methods of the invention allow the production of plants and seeds having expression of one or more desired genes in one selected tissue of the plant. Thus, the methods of the invention permit the production of plants having one or more desired traits limited to selected plant tissues, thereby enabling the targeting of a trait to the tissue, or avoiding the expression of a desirable gene in a tissue where its effects are unwanted. There are a wide variety of specific applications of the invention, including, but not limited to, the production of plants having increased stress tolerance, having increased resistance to herbicides or pesticides, having improved nutrient uptake and/or utilization, having improved nutrient content and/or yields of desired compounds, having increased resistance to pests or disease, and having phytoremediative properties. Specific applications of the invention are further described below.

Environmental stresses, including drought stress, saline stress, light stress, pH stress, temperature stress, and oxygen stress, have a significant impact on the ability of plants to grow and thrive. Many soils are nonpermissive for growth of desirable plants due to one or more of the aforementioned environmental stresses. By the methods of the invention, it is possible to produce plants and seeds of plants which have improved tolerance to such stresses, thereby enabling growth in environments which normally would not support the growth of the plant. For example, using a root-specific plant promoter, it is possible to produce plants having additional transporter molecules to improve uptake of water from the surrounding soil, thereby improving growth of the plant in drought conditions. Similarly, the combination of a root specific promoter and genes encoding transport molecules may improve secretion of salts and alkaline or acidic compounds to the soil surrounding the plant, thereby permitting growth under saline stress or in alkaline or acidic soil. The use of leaf-specific promoters in combination with modified photosynthetic apparatus genes may permit the production of plants able to photosynthesize under different light conditions, thereby relieving light stress.

Plants are exposed to numerous pests, including insects, nematodes, fungi, bacteria and viruses. Soils infested with any of the above diseases or pests have traditionally not been able to support the growth of valuable crops and desired plants. Further, treatment of such diseases or removal of such pests has required measures such as application of pesticides, which may be detrimental to the growth of the plant, as well as harmful to the surrounding environment or the end-user of the plant. By the methods of the invention it is possible to produce plants expressing pest and disease-resistance genes where they are most needed to defend the plant against attack from diseases and pests. For example, by engineering a plant with a construct combining a tissue specific promoter and one or more desired structural genes, it is possible to protect one or more tissues of the plant against consumption or attack by an insect (e.g., by strengthening the cell walls of the epithelial cells in the leaf, root, or stem such that leaf, root, or stem-destroying insects may no longer be able to damage or feed on these tissues). Further, it is possible, using the methods of the invention, to specifically express plant immunity proteins (e. g., the N, M, L6,RPP1, and RPP5 genes of monocots, and the RPS2, RPM1, 12, Mi, Dm3, Pi-B, Xa1, RPP8, RPS5 genes of both monocots and dicots (see, e.g., Meyers et al. (1999) Plant J: 20(3): 317-332) in tissues typically invaded by bacteria and/or viruses (roots).

Aside from the aforementioned protective or prophylactic expression of structural or immunity proteins in specific desired tissues of the plant, it is also possible to express compounds and proteins which actively eradicate insects and other pests from the plant. For example, toxic compounds such as bacterial toxins or enzymes which specifically target essential insect proteins or membrane constituents of plant pathogens (e.g., delta-endotoxin from *Bacillus thuringiensis* (Parker and Luttrell.(1999).J. Econ. Entomol. 92 (4): 837-845) and chitinase (Ding et al. (1998) Transgenic Research 7(2): 77-84)) may be expressed in the roots, stems, or leaves of a plant to specifically target a pest known to attack the plant in one of these tissues. Simultaneously, the expression of the selected toxic compound in other tissues, such as the fruit or flower of the plant, is avoided, thereby limiting exposure of the end consumer of the plant to the chemical or protein. Further, the ability to engineer tissue-specific defensive and the active resistances to pests and microbes afforded by the instant invention offers the potential to reduce the necessity for pesticide use in pest and disease control, thereby resulting in less pollution of the environment and fewer unwanted side effects on other species of plants and animals.

Another application of the invention is in the production of plants better able to thrive on nutrient-poor soils. It is well known in the art that certain plant species, particularly crop plants, deplete the soil of nutrients necessary to sustain growth, such as nitrogen, phosphate, and potassium. In order to replenish the lacking nutrients, it is necessary either to fertilize the soil (an expensive and environmentally damaging procedure) or to cultivate plants known to deposit the depleted nutrient into the soil (e.g., clover or soybean in the case of nitrogen depletion), which may be less economically or nutritively desirable crops. The methods of the invention permit the targeted expression in the root of genes involved in nutrient uptake (e.g., transport molecules) to those tissues in which the uptake occurs to thereby improve the ability of the plant to absorb the nutrient from the environment. The invention may also be used to produce plants which express heterologous or optimized native nutrient utilization genes in the root that permit more efficient use of the nutrient, such that less of the nutrient is required for the normal growth and functioning of the plant. Further, it is possible, using the methods of the invention, to express in the root genes involved in the use and uptake of nutrients not normally used by the plant in those plant tissues which are directly exposed to the different nutrient. In this fashion, plants which are able to grow and thrive on different nutrient sources (e.g., different nitrogen sources) may be produced. Particularly useful target genes for the optimization of nitrogen efficiency of the plant include: glutamine synthetase (GS), asparagine synthetase (AS), glutamate synthase (also known as glutamate 2: oxogluturate amino transferase and GOGAT), asparaginase (ANS), glutamate dehydrogenase (GDH), alanine dehydrogenase, aspartate aminotransferase (AspAT) (activities are shown in Figure I) and alanine aminotransferase (AlaAT) (activity shown in Figure 2).

Further, the methods of the invention permit the production of plants with altered nutritive content (e.g., protein content, sugar content, vitamin or mineral content, oil content, lipid content, carbohydrate content, starch content, or phytochemical (e.g., bioactive plant compounds such as steroids, anticancer compounds, stimulants, psychoactive compounds or other compounds having pharmaceutical properties) in the root. The selective expression of a vitamin synthesis protein in tuber or fruit tissue may result in fruit or tubers with increased vitamin content. Similarly, the selective expression of enzymes involved in sugar production in tuber or fruit may increase the sweetness of these tissues, making them more attractive for consumption or increasing the nutritive value of the tissue. Similarly, for those plants from which one or more desired compounds are extracted, being able to increase the production of the desired compound (e.g., a sugar (as in sugar cane) or other desirable plant compound (such as cacao or plant compounds with pharmaceutical properties)) in tissues of the plant from which the compound is readily extracted (e.g., leaf tissue or root tissue) may increase the yield of the desired compound from the plant.

Another application of the invention is in the production of plants which are resistant to herbicides and/or pesticides. Both herbicides and pesticides are commonly used to permit the continued growth and development of plants under conditions of pest or other plant infestation. By producing a plant which specifically expresses a protein which is able to detoxify the herbicide or pesticide (e.g., a transport molecule which permits specific excretion of the herbicide or pesticide, or an enzyme which converts the herbicide or pesticide into a harmless compound) in those plant tissues with the greatest exposure to the compound (e. g., the root of the plant), it is possible to produce plants which are resistant to the effects of the pesticide or herbicide.

The invention further enables the production of plants which may detoxify unwanted compounds (e.g., hydrocarbons or heavy metal wastes) in the environment (e.g., in soil, water, or air) and thus are useful in phytoremediation. For example, transgenic plants expressing a protein which is able to chelate heavy metals or to enzymatically convert a potentially harmful compound (e.g., a hydrocarbon such as petroleum or benzene) to a more benign degradation product would be useful and cost-effective in efforts to clean hazardous waste sites.

The invention further enables the use of plants in the production of desirable chemicals or proteins. In many instances, the synthesis of a desired chemical or protein is not readily feasible, and the isolation of the compound from natural sources is an expensive, unwieldy, or time-consuming process. Using the methods of the invention, it is possible to engineer a selected plant such that the plant expresses one or more selected genes (e.g., genes in which the expression product is desirable, or genes encoding enzymes which are involved in the production of the desired compound) in the root (e.g., those plant tissues from which extraction and subsequent purification of the compound is simplest). Examples of compounds which may be advantageously produced by application of the methods and compositions of the invention include, but are not limited to, vitamins, enzymes, amino acids, sugars, lipids, carbohydrates, and compounds having pharmaceutical properties, such as stimulants, anti-cancer compounds, and the like.

Selective inhibition of gene expression in one or more selected plant tissues may be achieved through the use of antisense nucleic acid sequences. Antisense nucleic acid technology is well known to one skilled in the art; for a general discussion of antisense technology in plants see, e.g., Shewmaker et al., U.S. Patent No. 5, 453, 566. Nucleic acid sequences whose transcription products are mRNA antisense to a target gene or genes may be expressed only in those plant tissues in which expression of the target gene or genes is undesirable. Upon transcription of the introduced nucleic acid in the selected tissues, the resulting antisense mRNA hybridizes to the mRNA of the target gene(s), thereby preventing the target gene mRNA from being translated. This may be useful, for example, in structurally altering certain tissues of the plant such that chemicals such as pesticides may be more readily taken up by the plant, or in altering the nutritive properties of the plant, for example by inhibiting the expression of one or more toxic compounds in the fruit of the plant. Other useful applications will be apparent to the skilled practitioner.

The genetic constructs described herein can also provide transformed plants which can express a desired gene when it is most needed or beneficial. Because the promoter from btg-26 from *Brassica napus* as discussed in Example 1 and shown in Figure 3 and SEQ ID NO:1, is stress inducible, when the target gene is a nitrogen assimilation and/or use gene, the plant is induced to assimilate and/or metabolize nitrogen upon application of the stress. Such a plant thereby can have increased stress tolerance, such as by enhanced osmoregulation. Other applications will be readily apparent to one skilled in the art.

As another example, where the promoter is induced by the presence of nitrate, such as, for example, the nitrate reductase promoter (Cheng *et al.*, 1988, *ibid.;* Cheng et al., 1991, Plant Physiol. 96:275-279), the plant will be induced to assimilate and/or use nitrogen upon application of a nitrogenous fertilizer. Alternately, or in addition, the promoter can be inducible, for example, by an exogenously applied chemical such as alcohol or ABA (see, e.g., Marcotte et al., 1989, Plant Cell 1 :969-976). This chemical could be included in nitrogenous fertilizer. Thus, plants can more efficiently utilize fertilizer input by rapidly taking up the nitrogen in the fertilizer and storing it at the time of application, to thereby reduce the amounts of nitrogenous fertilizer which are lost to leaching, etc. This may permit a reduction in the amount of nitrogenous fertilizer required to be applied to a crop, to obtain crop yields comparable to those obtained using normal cultivation techniques and plants which have not been modified according to the present invention. Additional agronomic advantages can include faster growth and crop yield, where nitrogenous fertilizer input is maintained at levels used in common crop cultivation techniques.

Transformed *Brassica napus* plants ectopically expressing a gene encoding an enzyme involved in nitrogen assimilation or metabolism, for example but not limited to alanine dehydrogenase, glutamine synthetase, asparagine synthetase, glutamate synthase, asparaginase, glutamate dehydrogenase, aspartate aminotransferase, and alanine aminotransferase (Figures 1 and 2) were grown under field conditions (Example 6) to determine if the beneficial effects on plant growth and yield under controlled growth ' conditions (Examples 3 and 4) would be retained. For example, it was examined whether-the amount of available nitrogen would have an effect on plant biomass and seed yield on plants expressing btg-26/AlaAT grown under field conditions. However, it is to be understood that plants grown under field conditions are subjected to a range of environmental adverse conditions including salinity stress, temperature stress, nutrient stress, pH stress, drought stress, heavy metal stress and the like, and while the amounts of nitrogen may be varied, these plants will also be exposed to other environmental stresses.

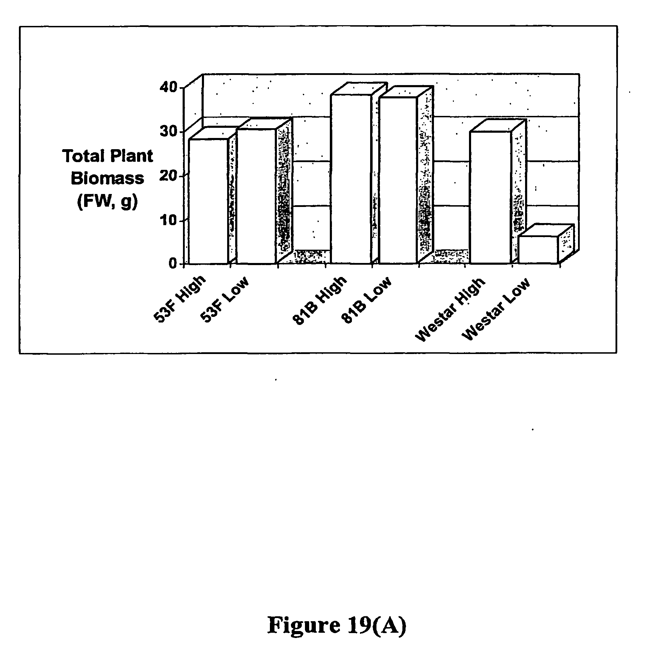

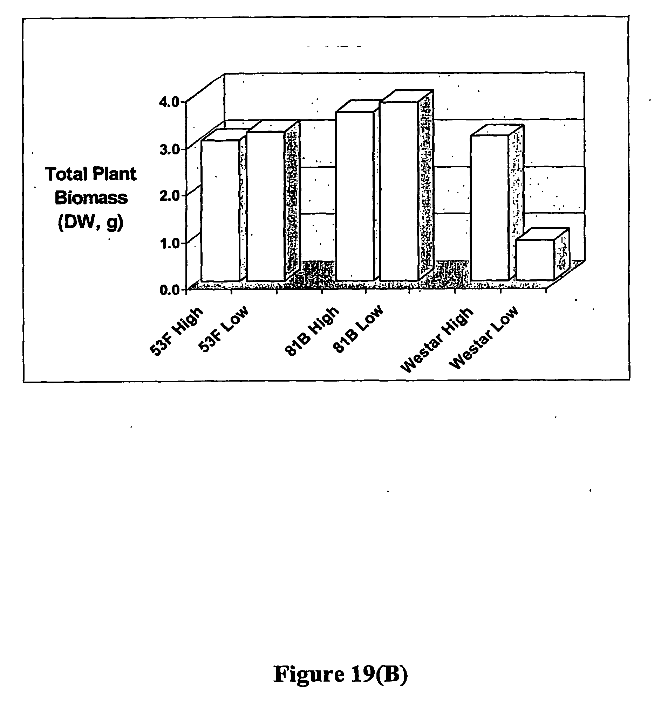

As can be seen with reference to Figure 19 A, transgenic plants expressing a gene encoding an enzyme involved in nitrogen assimilation or metabolism, for example but not limited to AlaAT (plants 53F and 81B), exhibited a similar yield in plant biomass irrespective of the nitrogen availability. However, control plants exhibited lower yields under conditions of low nitrogen. Furthermore, the plant biomass observed in the transgenic plants in the presence ("high") or absence ("low") of additional N, were equivalent to the control plants receiving N ("high"). These results show that ectopic expression of a gene encoding an enzyme involved in nitrogen assimilation or metabolism results in increased plant biomass under a range of environmental conditions, including variable nitrogen availability. In the absence of any added nitrogen, the transgenic plants produced higher yields than control plants indicating that the transgenic plants were better able to grow under environmentally adverse conditions.

Therefore, a method for increasing biomass of a plant growing under one or more environmentally adverse conditions, such as conditions of low nitrogen may comprise;
transforming a plant with a target gene in operative linkage with a promoter element to produce a transformed plant, the target gene encoding an enzyme involved in nitrogen assimilation; and growing the transformed plant.

Seed yield in control and transgenic plants grown under field conditions was examined (Figure 19B). Control plants produced higher seed yields when supplied with additional nitrogen. However, transgenic plants expressing a gene encoding an enzyme involved in nitrogen assimilation or metabolism exhibited higher yields than control plants in the presence or absence of additional N. Also shown in Figure 19B, is that the yields observed with the transgenic plants were equivalent in plants grown on either high or low nitrogen. Collectively these results indicate that transgenic plants ectopically expressing an enzyme involved in nitrogen assimilation or metabolism are capable of optimising the utilization of available nitrogen under a range of environmental conditions thereby resulting in an increase in plant biomass, seed yield or a combination thereof.

Therefore, a method for increasing seed yield of a plant may comprise;
transforming the plant with a target gene in operative linkage with a promoter element, to produce a transformed plant, the target gene encoding an enzyme involved in nitrogen assimilation or metabolism; and growing the transformed plant.

The above description is not intended to limit the claimed invention in any manner, furthermore, the discussed combination of features might not be absolutely necessary for the inventive solution.

The present invention will be further illustrated in the following examples. However it is to be understood that these examples are for illustrative purposes only, and should not be used to limit the scope of the present invention in any manner.

### EXAMPLES

### Example 1: Isolation and Characterization of Osmotic Stress-Induced

### Promoter

A *Brassica napus* (cv. Bridger) genomic DNA library (Clontech, Palo Alto, California) was screened using standard techniques (Ausubel et al., 1989, Current Protocols in Molecular Biology, Wiley, Wiley: N. Y.) with the Pisum sativum 26g cDNA (complementary deoxyribonucleic acid) clone (Guerrero et al., ibid), ³²P-labelled with a Random Primer Kit (Boehringer Mannheim, Laval, Quebec). A 4.4 kb Sall fragment containing the entire *btg-26* gene was subcloned into the commercially available pT7T3-19U vector (Pharmacia Canada, Inc., Baie d'Urfe, Quebec, Canada) for further analyses.

### Identification of a osmotic stress-induced promoter in Brassica napus

Several genes activated during drought stress have been isolated and characterized from different plant species. Most of these represent later-responding, ABA-inducible genes (reviewed by Skiver and Mundy, *ibid.).* Recently, however, an ABA-independent, cycloheximide-independent transcript, 26g, was reported in *Pisum sativum* (Guerrero and Mullet, *ibid*; Guerrero *et al., ibid).* Because this gene does not require protein synthesis for activation, it is postulated that it represents an early factor in the drought signal transduction pathway. To isolate an osmotic stress induced promoter from *Brassica napus,* the cDNA clone representing the *P. sativum* 26g gene (Guerrero *et al., ibid)* was used. Total RNA was isolated from the third leaf of whole plants that had been either watered continuously or dehydrated for four days. Using low stringency hybridization, RNA blot analysis identified a single 1.75 kb transcript that is greatly induced in droughted plants (data not shown). To determine if this mRNA represents a single copy gene in *B. napus,* genomic DNA was digested with EcoRI, HindIII or BgIII and analyzed by DNA blot hybridization using the *P. sativam* 26g cDNA. A single band was identified in each lane (data not shown). It was concluded that this transcript represents a single copy, drought-induced gene in *B. napus.* This gene is referred to as *btg-26 (Brassica* - turgor gene - 26).

### Structure of the btg-26 gene

To isolate the btg-26 gene, a *B. napus* genomic DNA library in EMBL-3 (Clontech, Palo Alto, California) was screened with the P. *sativum* 26g cDNA. From 40,000 plaques analyzed, a single positive clone was identified with an insert size of approximately 16 kb. A 4.4 kb SalI fragment containing the entire gene was subcloned. The promoter sequence of the btg-26 gene was determined by identification of the mRNA start site using primer extension (Ausubel, *ibid.)* and is shown in Figure 3 and SEQ ID NO: 1. In Figure 3, the transcription start site is bolded, underlined and indicated by +1. The TATA box and CAAT box are in bold and double underlined. Postulated functional regions are underlined. The sequence of the btg-26 promoter, coding region and 3' region has been presented in Stroeher et al, (1995, Plant Mol. Biol. 27:541-551).

### Expression analysis of btg-26

Induction of btg-26 expression during droughting was examined by RNA blot analysis. Potted *B. napus* plants were naturally dehydrated by withholding water for various lengths of time. Whole leaves were used either to determine relative water content (RWC) of individual plants or to isolate total RNA. As shown in Figures 4A and 4B, btg-26 expression is induced rapidly during water loss, reaching a six-fold increase over expression in fully hydrated plants at 81% RWC, increasing to eleven-fold induction - at 63 % RWC. Further decreases in RWC were associated with a decrease in total amount of *btg-26* transcript. At 30% RWC expression was only 3.5-fold over fully hydrated levels.

Because other physiological stresses alter intracellular water content, *btg-26* expression was examined in *B. napus* plants exposed to cold, heat shock and salt stress. RNA blot analysis indicated that there was no change in *btg-26* expression when plants were transferred from normal growth conditions to 4°C for one day. However, plants left at 4°C for four days showed a five-fold induction in *btg-26* mRNA. A similar increase was seen when plants were shifted to 40°C for two or four hours. These results are shown-in Figure 4C and demonstrate that expression of *btg-26 is* induced during temperature stress. To examine the effect of salt stress, plants were watered to capacity one day or four days with 50 mM, 150 mM, or 450 mM NaCl. The level of *btg-26* expression was not affected by 50 mM NaCl regardless of length of exposure. However, growth in 150 mM NaCl caused a two-fold increase in *btg-26* mRNA after four days. Exposure to 450 mM NaCl caused the most notable induction, twelve-fold after one day, dropping to four-fold after four days. Refer to Figure 4D for Northern blots showing these results.

Finally, many drought-inducible genes are also ABA responsive. To examine the role of ABA in *btg-26* expression, total RNA was isolated from individual leaves treated with or without ABA. In these experiments, leaves were cut at the petiole and placed in a solution of 0 iM, 50 iM or 100 ìM ABA (mixed isomers, Sigma), 0.02% Tween-20 and pH 5.5 for 24 hours. As shown in Figure 4E, btg-26 expression is induced 2.5-fold when exposed to 100 iM ABA. However, when leaves were exposed to 50 iM ABA, no induction of expression was observed (data not shown). These results indicate that btg-26 is ABA responsive, but that this responsiveness is concentration dependent.

### Example 2: Creation of Drought-Induced Nitrogen Assimilation

### Constructs

This step involved the production of either constitutive or drought induced AlaAT constructs and the introduction of them into *Brassica napus* using *Agrobacterium* mediated genetic transformation. The approach of introducing specific sense or antisense cDNA constructs into plants to modify specific metabolic pathways has been used in a number of species and to modify a number of different pathways. (See Stitt & Sonnewald 1995 for a review; Ann. Rev. of Plant Physiol. and Plant Mol. Biol. 46:341-368). The AlaAT cDNA was introduced under the control of three different promoters: (1) The CaMV promoter which has been shown to be a strong constitutive promoter in a number of different plant species; (2) the btg-26 promoter described in Example 1 and (3) the trg-31 promoter which was isolated from tobacco by Guerrero and Crossland *(ibid.).* The-CaMV promoter should result in the constitutive overexpression of AlaAT whereas btg-26 and trg31 should induce over expression of AlaAT only under conditions of specific stresses, including drought stress.

### Plasmid Constructs

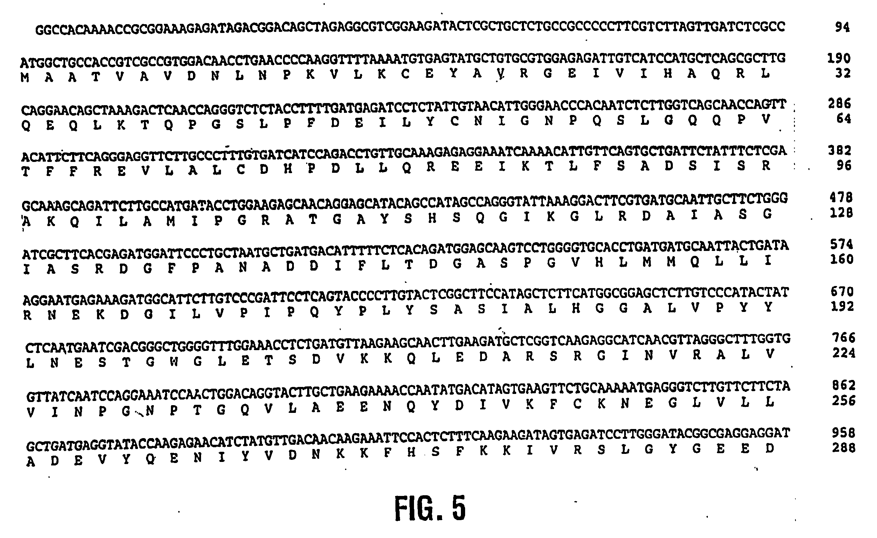

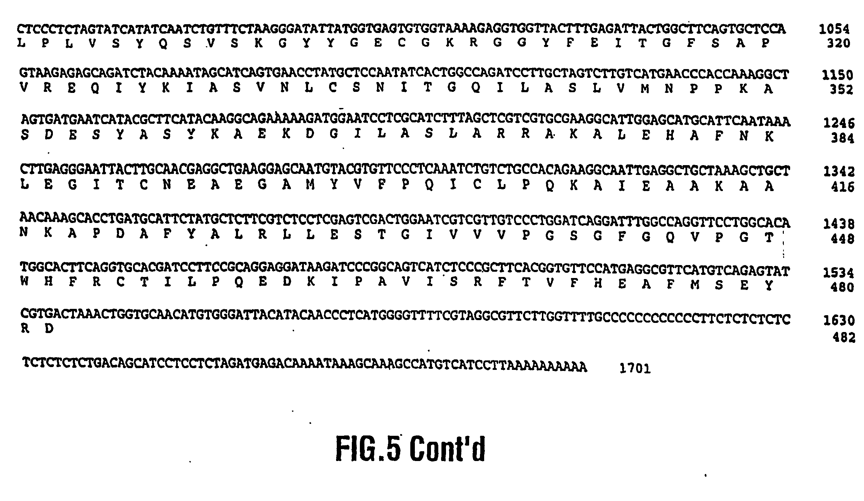

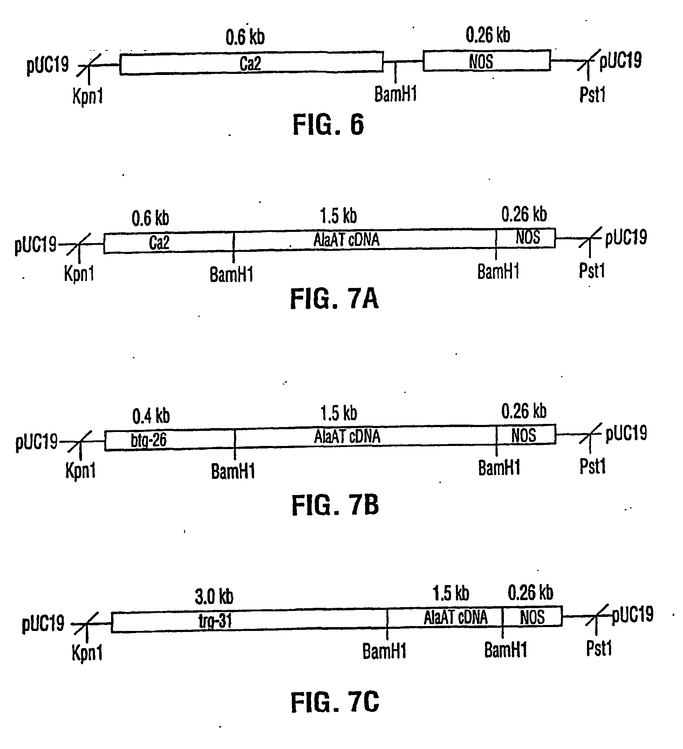

The barley AlaAT cDNA clone 3A (As shown in Figure 5 and Muench and Good, *ibid*) was cloned into the pT7T3-19U vector (Pharmacia Canada) and used for site directed mutagenesis using two specific primers. Primer 1 introduced a BamH1 restriction site between nucleotides 48-53, while primer 2 was used to introduce a second BamH1 restriction site between nucleotides 1558-1563 (See Figure 5). The 1510 bp fragment was then cloned into the vector p25 (Figure 6) which had been cut with BamH1. p25 was a gift of Dr. Maurice Moloney (Univ. of Calgary, Calgary, Alta., Canada). This construct contains the double CaMV35S promoter, which has been shown to give high constitutive levels of expression, and NOS terminator inserted into the Kpn1 and Pst1 site of pUC19 with a BamH1, Xbal and Pvul polylinkerbetween the CaMV and NOS region of the plasmid. The resulting plasmid was called pCa2/AlaAT/NOS, as shown in Figure 7A.

The plasmids ptrg-31/AlaAT/NOS and pbtg-26/AlaAT/NOS were created as follows. The trg-31 promoter was subcloned as a 3.0 kb Xbal/BamH1 fragment into the Xbal/BamH1 site of pCa2/AlaAT/NOS which had been digested with Xbal/BamH1 to release only the CaMV promoter, resulting in a 3 kb promoter fragment inserted in front of the AlaAT coding region. btg-26/AlaAT/NOS was created by inserting a BamH 1 site at nucleotides +9 to +14 (see Figure 3) and subcloning the 330 bp Kpnl/BamH 1 fragment (from -320 to +10 in Figure 3) into the Kpnl/BamH 1 site of pCa2/AlaAT/NOS which had been digested to release the CaMV promoter. Plasmid constructs pbtg-26/AlaAT/NOS and ptrg-31/AlaAT/NOS can be seen in Figures 7B and 7C, respectively.

### Transformation and analysis of Brassica napus plants with AlaAT constructs

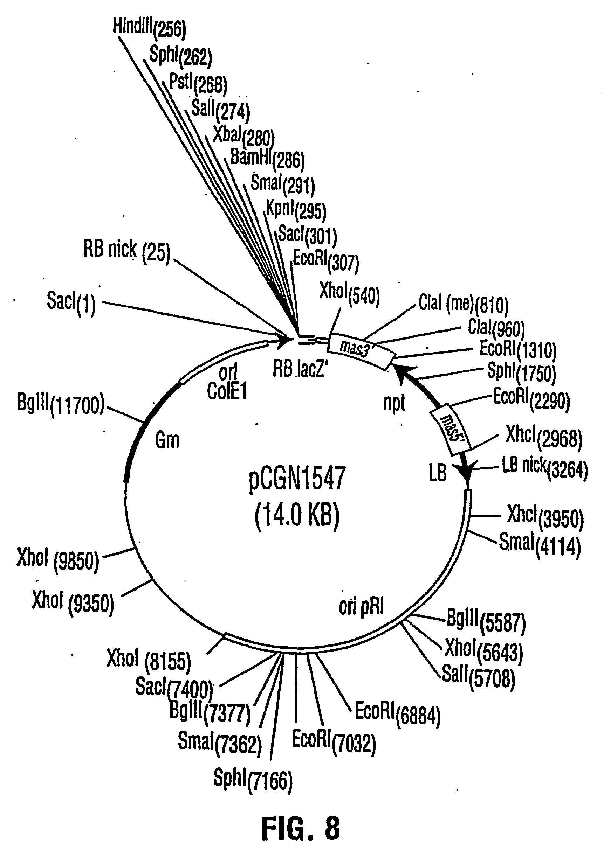

Once the three plasmids, as shown in Figures 7A, 7B and 7C, containing the AlaAT gene had been confirmed by restriction analysis and sequencing they were subcloned into the transformation vector pCGN1547 (Figure 8). pCGN1547 is an *Agrobacterium* binary vector developed by McBride and Sunlmerfelt (1990, Plant Mol., Biol. 14:269-276)*.* pCGN1547 contains the neomycin phosphotransferase II (NPTII) gene which encodes Kanamycin resistance. These constructs were then introduced into the *Agrobacterium* strain EHA101 by electroporation using the protocol of Moloney et al. (1989, Plant Cell Reports 8:238-242). Confirmation that the *Agrobacterium* had been transformed with the pCGN 1547 vector containing the specific construct was confirmed by polymerase chain reaction (PCR).

Transgenic plants were produced using a cotyledon transformation approach as described by Moloney *et al. (ibid.).* Kanamycin resistant plantlets were transferred to soil and then grown. The initial generation, or primary transformants, were referred to as the T0 generation and were allowed to self. Each subsequent generation was bagged to ensure selfing and referred to as the T1, T2 generation respectively. All putative T0 transgenic plants were tested for the insertion *of the Agrobacterium* construct using PCR primers that amplify the NPTII gene and by testing for NPTII activity as described by Moloney et al *(ibid.).*

### Analysis of Transformed Brassica plants containing the AlaAT constructs

Transgenic plants were assayed for AlaAT activity as follows. Extractions were carried out on ice as described previously (Good and Crosby, 1989, Plant Physiol. 90:1305-1309). Leaf tissue was weighed and ground with sand in a mortar and pestle in extraction buffer containing 0.1 M Tris-HCl (pH 8.5), 10 mM dithiothreitol, 15% glycerol and 10% (w/v) PVPP. The extract was clarified by centrifugation at 6,000 rpm and the supernatant was assayed for enzyme activity. AlaAT assays were performed in the alanine to pyruvate direction as described previously (Good and Crosby, *ibid*) using alanine to start the reaction.

After transformation 20 Ca2/AlaAT/NOS, 24 btg-26/AlaAT/NOS and 21 trg-31/AlaAT/NOS plants were produced which appeared to be transformed, based on the amplification of an NPTII PCR product and NPT activity. AlaAT activity was measured, using the method described above, in the leaf tissue of several of these transformants. As can be seen from Table 1, the btg-26/AlaAT/NOS plants had AlaAT activity levels that ranged from 1.63 to 3.89 times that of the wild-type, control plants. Ca2/AlaAT/NOS plants had activity levels that ranged from 1.51 to 2.95 times that of wild-type, control plants. Western blots confirmed that the transgenic plants had elevated levels of AlaAT, based on the cross reactivity of a band with the barley AlaAT antibody (not shown).

| Table 1: Alanine Aminotransferase (AlaAT) activity in Primary Transformants | |
|---|---|
| Plant | Activity* |
| Btg-26/AlaAT/NOS | |
| | |
| Transformant #4 | 3.89x |
| Transformant #5 | 1.63x |
| Transformant #7 | 1.93x |
| Transformant #8 | 1.98x |
| Transformant #18 | 1.63x |
| | |
| Ca2/AlaAT/NOS | |
| | |
| Transformant #1 | 1.51x |
| Transformant #2 | 2.77x |
| Transformant #6 | 1.61x |
| Transformant #7 | 2.95x |
| Transformant #9 | 2.14x |
| Transformant #12 | 1.91x |
| Transformant #13 | 1.77x |
| * Enzyme activity is expressed relative to wild-type controls | |

### Example 3: Growth of Primary Transformants Under Normal Conditions

T1 seed from the primary transformants of the groups CaMV/AlaAT and btg-26/AlaAT were grown along with control, wild-type plants under normal conditions including planting at a 1 cm depth in 13 cm diameter plastic pots containing a soil and fertilizer mixture as described by Good and Maclagan (*ibid*.). These pots were placed in growth chambers under the following conditions: i) 16 h of 265 mmol m² s⁻¹ provided by VITA-LITE U.H.O. fluorescent tubes, ii) day and night temperatures of 21 °C and 15 °C-respectively, iii) relative humidity of 85%-97% and iv) daily watering with 1/2 strength Hoagland's solution. The only observable difference observed between the plants was the btg-26/AlaAT plants had thicker stems when compared to the controls and CaMV/AlaAT plants. No significant differences were observed between the three groups in terms of growth rate, plant or leaf size or leaf senescence at identical time points, time to maturity, seed size or seed yield.

### Example 4: Growth of Primary Transformants Under Nitrogen-Starved/Drought Conditions

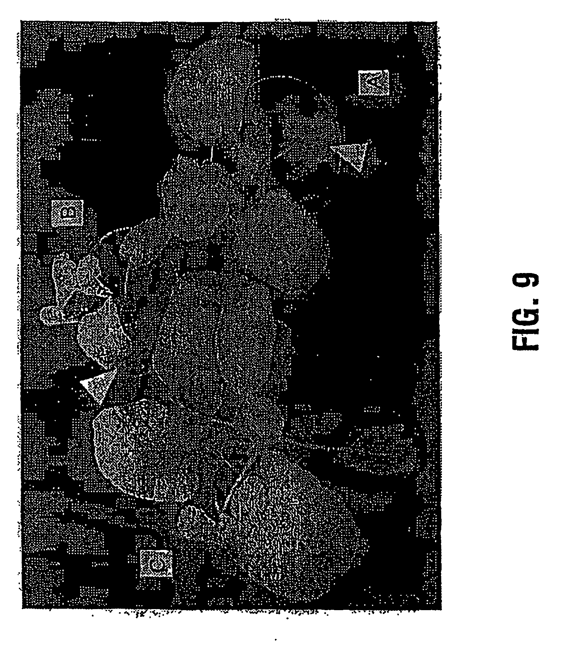

T1 seed from the primary transformants of the CaMV/AlaAT and btg-26/AlaAT groups were grown along with control, wild-type plants for four weeks under normal conditions (as noted above) and then subjected to nitrogen starvation, by watering with only water for three weeks, followed by drought for 3 days. Figure 9 shows representative plants from the three groups after the treatment at identical time points. Plant A is a control, wild-type plant; Plant B is a CaMV/AlaAT transformed plant; and Plant C is a btg-26/AlaAT plant. It can be seen that plant C (btg-26) clearly has a faster growth rate than plants A (control) and B (CaMV/AlaAT). In addition, senescing leaves (indicated by arrows) are present on plants A and B while plant C has no senescing leaves. In summary, the following were observed in the treated btg-26/AlaAT plants when compared to the treated CaMV/AlaAT and control plants: faster growth rate; larger plants at similar time points, less senescence in the lower leaves; earlier maturity; thicker stems; larger seeds; and higher seed yields.

### Example 5: Tissue-Specific Expression of Genes Utilizing the btg-26 Promoter

To determine whether genes under the regulatory control of the btg-26 promoter were expressed in a tissue-specific manner, experiments were performed in which the levels of the expressed protein product of a transgene placed under the control of the btg-26 promoter were measured in either the shoot or root of a transgenic plant containing the btg-26 construct. Both a reporter gene (GUS) and a functional gene of interest (AlaAT) were utilized, and the expression of their respective protein products in either the shoot or root of transgenic plants was determined both qualitatively and quantitatively.

### Production of btg-26/GUS transgenic plants

Plants expressing the reporter gene GUS under the regulatory control of the btg-26 promoter were produced. First, a btg-26/GUS plasmid was created by inserting a 300 bp Kpnl/BamHI btg-26 promoter fragment into the Kpn1/Bam HI site of pBI101 which had been digested to release the CaMV promoter. This plasmid was then subcloned into the transformation vector pCGN1547 which was introduced into the Agrobacterium strain EHA101 by electroporation using the protocol of Moloney *et al.* (*ibid*)*.* Confirmation that the Brassica plants had been transformed was obtained by a) PCR amplification of the NPTII gene coding for neomycin phosphotransferase and by b) testing for NPTII activity as described by Moloney *et al.* (*ibid*)*.* Transgenic plants (T0) were allowed to self and then T1 plants selfed to produce T2 seed. The T2 seed was tested using a Kanamycin resistance assay (Moloney *et al.* 1989, *supra)* to ensure the seed was homozygous.

### GUS staining of btg-26/GUS transgenic lines in vivo

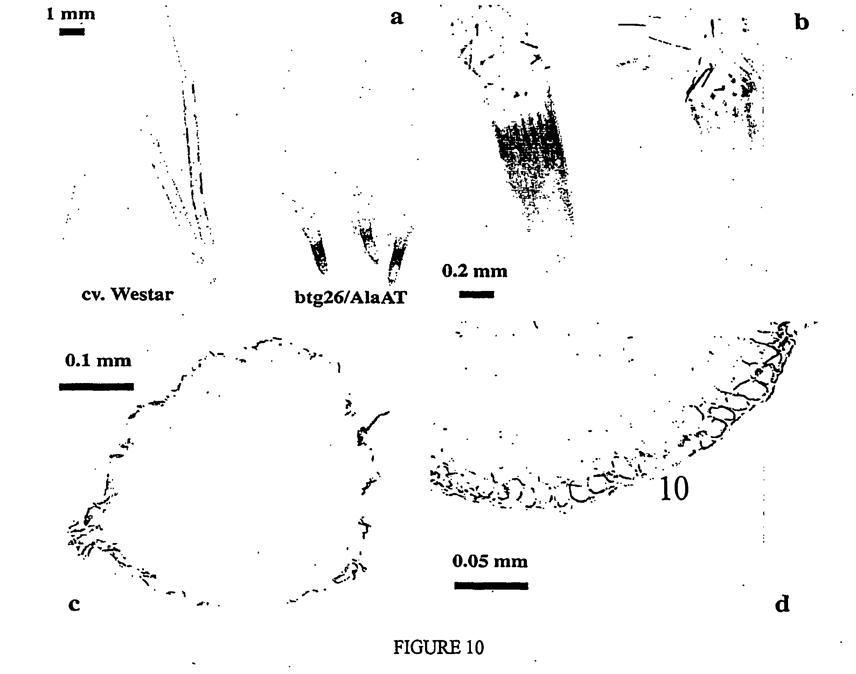

To determine the tissue distribution of expression of the GUS gene from the btg-26/GUS construct within the transgenic plants described in the previous section, the activity of GUS in different tissues was ascertained by the utilization of a colorimetric reaction, the results of which could be visually assessed. Plants were germinated and grown hydroponically in sterile conditions in modified Long Ashton media (Savidov *et al.* 1998) in Magenta jars, which were bubbled with air. Five week old plants were stained for *in vivo* GUS activity by replacing the MS media with 50 mM phosphoate buffer (pH 7.5) containing 0.2 mM X-gluc (5-boromo-4-chloro-3-indolyl-beta-glucuronic acid) and incubating the plants in this media for 24 hours. Root tissue was then viewed under a dissection microscope at the magnification indicated and photos were taken. As shown in Figure 10, the btg-26 promoter directs expression of a reporter gene (GUS) in the root hairs (panel B), and a single layer of epidermal cells in the roots (panels C and D). Moreover, the promoter directs expression in the root tip, the cell division area and the area of cell expansion (panels A-D).

### GUS staining of btg-26/GUS transgenic lines in vitro

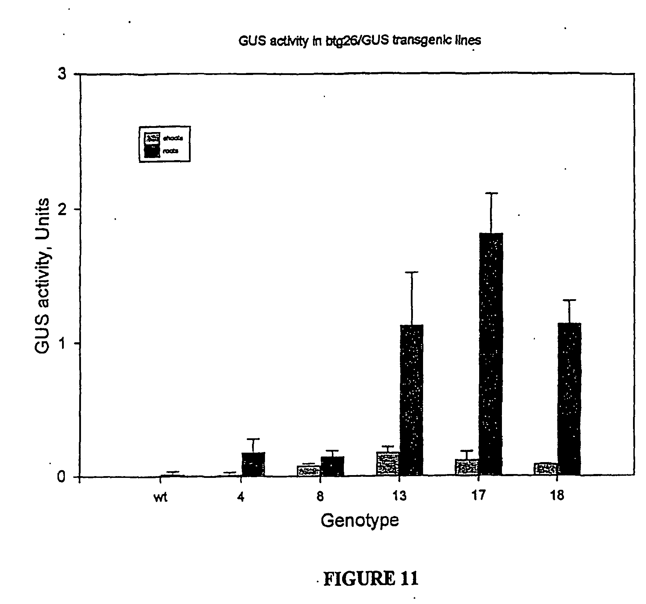

The above-described staining assay permitted a qualitative, visual analysis of the expression pattern of the btg-26/GUS construct in different tissues of the transgenic plant. To obtain a quantitative analysis of the tissue distribution of btg-26-directed expression, the levels of GUS activity in different tissues of the transgenic plant were also measured. Plants were grown as described above (*GUS staining of btg-26*/*GUS transgenic lines in* vivo), and the tissue was harvested and ground in GUS assay buffer. The *in vitro* GUS activity was measured as described by Gallagher, S.R. (1992) GUS Protocols: Using the GUS Gene as a Reporter of Gene Expression. Academic Press: New York,_ISBN 0-12-274010-6. As is shown in Figure 11, the btg-26 promoter directs expression of a reporter gene (GUS) in the root tissue significantly more strongly than in the shoot (leaf) tissue (between about two-fold and about 20-fold more strongly).

### Differential Expression of the AlaAT transgene in the roots and leaves of btg-26/AlaAT transgenic lines

To determine whether the btg-26/AlaAT construct described previously is expressed in a tissue-dependent manner similar to that of the btg-26/GUS construct, the expression of AlaAT in root and leaf tissue of transgenic plants was assessed by reverse transcriptase-PCR (RT-PCR). This methodology permits sensitive detection of the presence of AlaAT mRNA, and, coupled with densitometric methods, permits the quantitation of the translated product in a given tissue of the transgenic plant.

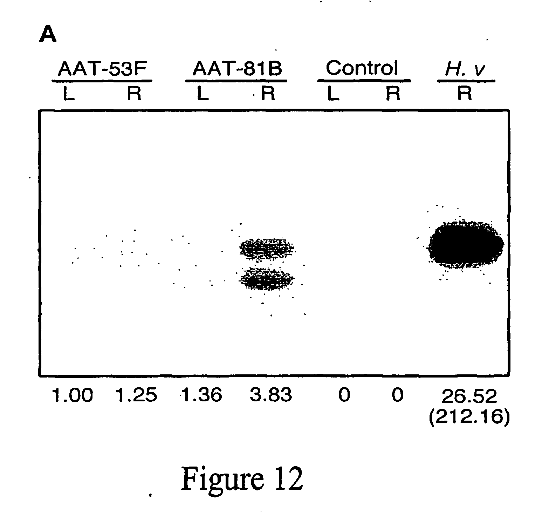

Differential expression of the AlaAT transgene in the roots and leaves of btg-26/AlaAT transgenic lines was confirmed using RT-PCR as per normal molecular protocols. Figure 12 is a southern blot analysis of RT-PCR products from leaf and root RNA. Leaf tissue was harvested from 5 week old plants grown as described above *(GUS staining of btg-26*/*GUS transgenic lines in vivo)* whereas root tissue was harvested from plants grown as described below (*Differential expression of the AlaAT transgene in the root tissue of the btg-26*/*AlaAT transgenic lines).* Primers amplified a 381 bp product at the 5' end of the AlaAT transgene and a 311 bp product which has no homology to the AlaAT cDNA. Based on the relative densitometry of the 381 bp product (indicated below each lane in Figure 12), it is apparent that the btg-26 promoter directs expression of the transgene preferentially (1.25 - 2.8 fold greater expression) in root tissue in those transgenic lines which display the N-efficient phenotype (and hence are known to be expressing the AlaAT protein product).

### Differential expression of the AlaAT transgene in the root tissue of btg-26/AlaAT transgenic lines

Visual confirmation of the above results was achieved by immunolocalization of the AlaAT protein product in root tissues of plants containing the btg-26/AlaAT construct. Plants were grown hydroponically in modified Long Ashton media (Savidov et al. 1998 Plant Sci. 133:33-45) in a growth chamber (18 degrees C, 350 uE,16 h light/8 hours. The roots were excised after 5 weeks and stained using an AlaAT antibody. Staining involved imbedding roots in paraffin, sectioning with a microtome and then using an AlaAT-specific antibody (Muench, D.G. and A.G. Good (1994) Plant Mol. Biol. 24:417-427) and a peroxidase goat anti-rabbit secondary antibody as per normal immunolocalization protocols.

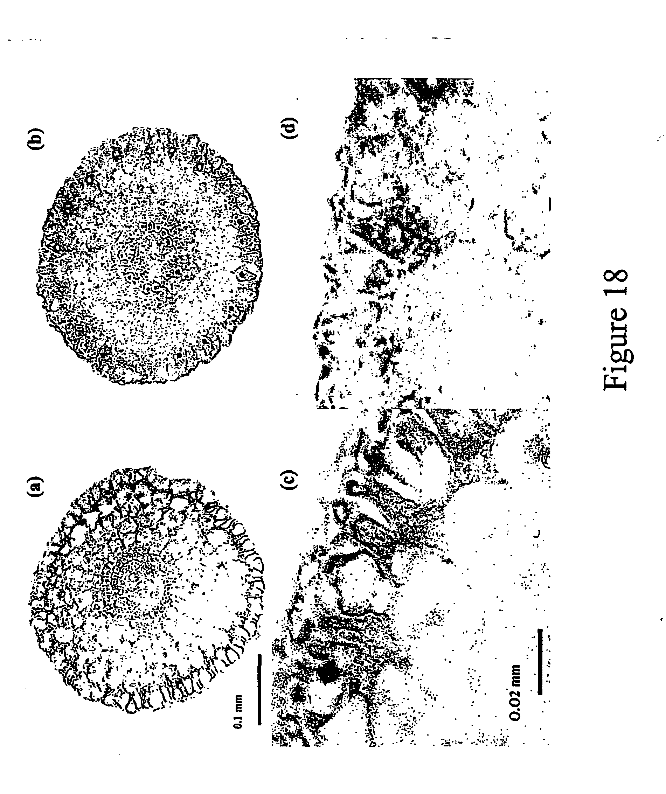

Specifically, tissues were fixed in FAA (50% ethanol, 5% acetic acid, 10% formalin), dehydrated in tert-butanol, and embedded in paraffin. Sections 10 microns in thickness were deparaffinized in xylenes, were subsequently rehydrated, and were blocked with PBS containing 3% non-fat dry milk for 3 h. The sections were mounted on glass slides that were coated with poly-L-lysine to promote adhesion. The antibody was diluted 1:100 in PBS, and this diluted antibody was incubated with the slide-mounted sections for 1 h. Post incubation, these tissue sections were washed extensively with PBS. Tissue sections were subsequently incubated for 1 h with anti-IgG secondary antibody (diluted 1:300 in PBS) conjugated to alkaline phosphatase. Color development was in AP buffer (100 mM Tris-HCl, pH 9.5, 100 mM NaCl, 5 mM MgCl₂) using 5-bromo-4-chloro, 3-indolylphosphate (BCIP) (0.005% [w/v] and nitroblue tetrazolium (NBT) (0.01%[w/v]) as substrates. Developed sections were dehydrated and mounted. As is shown in Figure 18, The btg-26/AlaAT plants expressed the AlaAT gene in a similar fashion to that seen in the btg-26/GUS transgenic plants; the tissue specific pattern of expression was identical (see Figure 18).

### Differential Expression of the AlaAT transgene in leaf tissue of the btg-26/AlaAT transgenic lines

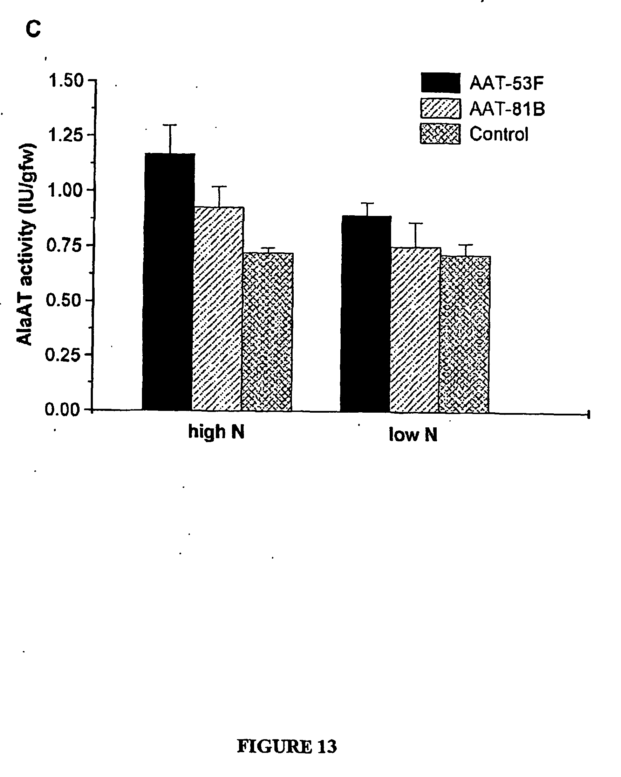

The tissue-specific expression of the AlaAT transgene in leaves of transgenic plants containing the btg-26/AlaAT construct previously identified by RT-PCR (see above) was confirmed by use of spectrophotometric assays measuring the enzymic activity of AlaAT in leaf extracts from transgenic and control plants. Plants were grown in a standard potting mixture (sand, peat moss, soil and slow release fertilizer, N/P/K), with or without supplementary nitrogen treatment. Plants were grown for 5 weeks in a growth chamber under standard conditions and FW, DW, leaf area and stem diameter were measured. Leaf proteins were extracted by grinding leaf tissue (0.2gm/ml) in a mortar and pestle in extraction buffer (0.1 M Tris-HCl, pH 8.0, containing 10 mM DTT and 10% (v/v) glycerol). AlaAT activity was assayed spectrophotometrically essentially as described (Good and Muench, *supra).* The reaction mix contained 5 mM 2-oxoglutarate, 0.1 mM NADH, 100 mM Tris-HCl (pH 8.0), 5 units of LDH (Sigma L1254), and 20 iL of enzyme extract to a final volume of 1 mL. The reaction was started by the addition of 25 mM alanine, and the absorbance change was measured at 340 nm at 21 °C. As is seen in Figure 13, the btg-26/AlaAT transgenic plants have enhanced levels of AlaAT activity in leaf tissue.

### Differential induction of the AlaAT transgene in shoots and roots using salt

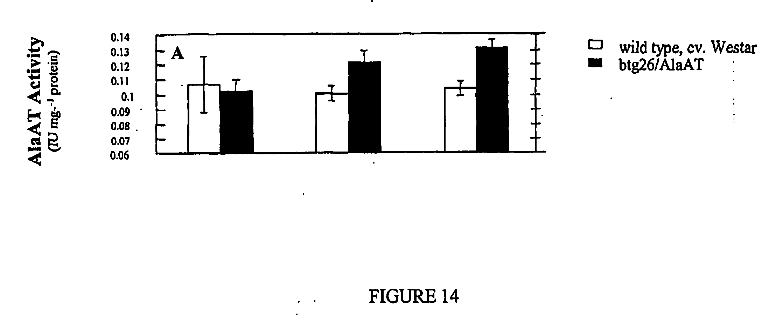

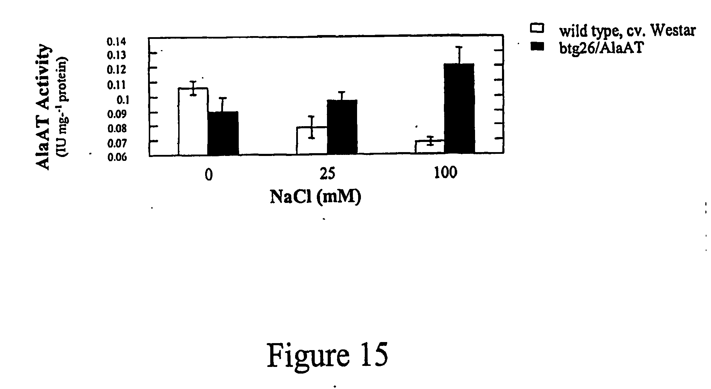

It had been demonstrated (Example 1) that the expression of genes under the control of the btg-26 promoter could be switched on by treatment with NaCl, in a concentration-dependent manner. To determine whether the tissue-specific expression of AlaAT in plants transgenic for the btg-26/AlaAT construct could also be effected by a saline treatment, the following experiment was performed. Plants were grown hydroponically in a modified Long Ashton's nutrient solution containing 0.5 mM nitrate within growth chambers in 60 liter tanks. After 4 weeks of age, differing concentrations of salt were added to the media and the level of AlaAT activity was measured 36 hours : after the addition of NaCl, as described by Good and Muench (1992, *ibid).* The results are shown in Figures 14 and 15. Whereas in wild-type shoots or roots, saline-treated plants display decreased AlaAT activity from the untreated controls, the saline-treated btg-26/AlaAT transgenic shoots or roots both display significant increases in AlaAT activity over untreated controls (see, e.g., Figure 18). This demonstrates that the expression of the AlaAT gene can be switched on in root and shoot tissue by the addition of an inducing compound, in this case NaCl.

### Differential expression of the AlaAT transgene, when induced by salt, results in enhanced growth rates compared to the untransformed controls

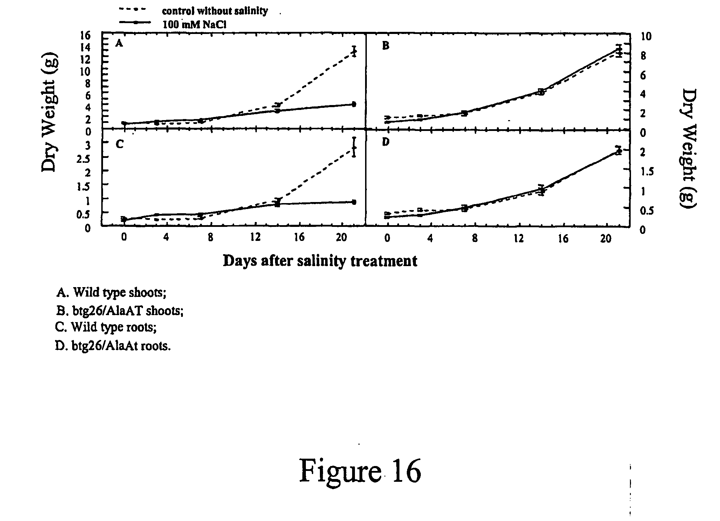

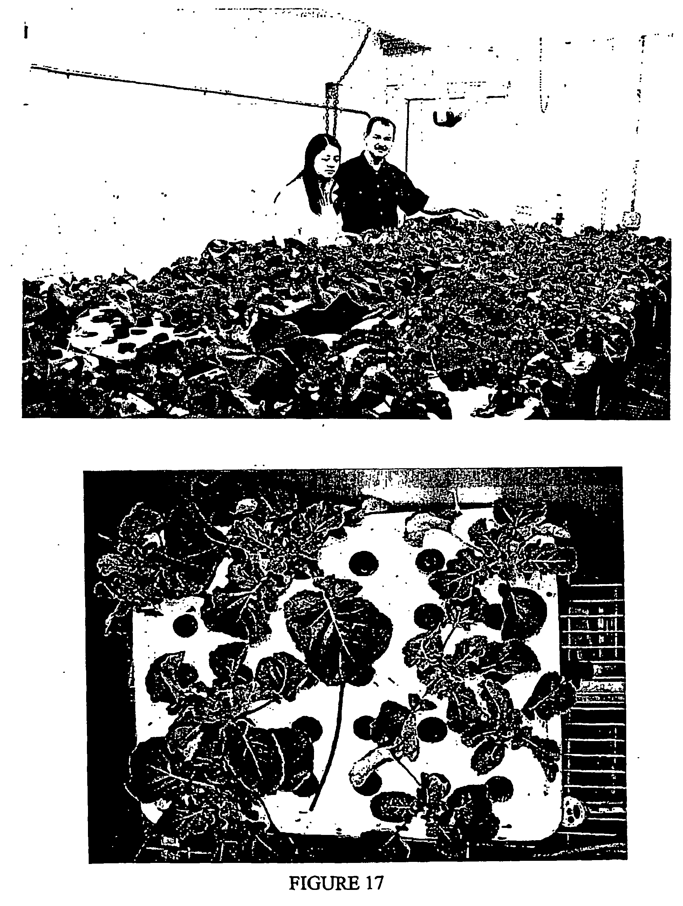

It had been previously ascertained (Example 4) that plants carrying the btg-26/AlaAT construct were visually improved in growth over control plants under conditions ofnitrogen starvation or drought. The above-recited results demonstrated that AlaAT expression as directed by the btg-26 promoter is tissue-specific not only to shoots (see Figure 14), but also to roots (see Figure 18). Experiments were performed to quantitatively determine whether direct treatment of transgenic plants containing the btg-26/AlaAT construct with NaCl results in a similar growth effect in one or both of the plant tissues demonstrated to have significant AlaAT expression (e.g., shoot and root). Plants were grown hydroponically in growth chambers in 60 liter tanks (As described above) in a modified Long Ashton's nutrient solution containing 0.5 mM nitrate. After 4 weeks of age, differing concentrations of salt were added to the media and the fresh weight and dry weight of the roots were measured. As is shown in Figure 16, there is enhanced growth (as measured in biomass) in plants expressing the AlaAT gene in root tissue (panels B and D) by the addition of an inducing compound (such as NaCl) as compared to wild-type control plants under similar conditions (panels A and C). This growth effect is sufficiently significant as to be visually apparent (Figure 17); the btg-26/AlaAT plants treated with 100 mM NaCl for 2 weeks after 4 weeks of age shown on the right side of the figure are visually improved in growth over the wild-type plants treated under the same conditions, shown on the left side of the figure.

### Example 6: Effect of expressing btg-26/AlaAT in plants, on plant biomass and seed. yield.

Transformed *Brassica napus* plants comprising btg-26/AlaAT were prepared as described in Example 2. Transgenic and native plants (Westar) were planted within plots using standard agronomic practice and grown under field conditions in the presence or absence of added nitrogen. At the time of planting the soil contained 35 to 50 lbs of available nitrogen/acre. The control group of plants (indicated as "low" in Figures 19 A and B) were fertilized with 20 lbs/acre potassium (K), 45 lbs/acre phosphorous (P) and 0 lbs /acre nitrogen (N). The treated plants (indicated as "high" in Figures 19 A and B) were fertilized with 20 lbs/acre potassium (K), 45 lbs/acre phosphorous (P) and 100 lbs /acre nitrogen (N). Plant material obtained at the bolting stage was used for the determinations of total plant biomass (fresh and dry weights were determined). Seed yield was determined on a per plot basis.

As can be seen with reference to Figure 19 A, total plant biomass increased in the control plants in the presence of "high" nitrogen when compared with plants that did not receive any additional N ("low" in Figure 19A). However, transgenic plants expressing btg-26/AlaAT (53F and 81B), exhibited similar yields irrespective of the N availability. Furthermore, the yields observed in the transgenic plants in the presence ("high") or absence ("low") of additional N, were equivalent to the control plants receiving N ("high"). Figure 19A shows results of plant biomass on a dry weight basis, similar results were observed on a fresh weight basis. These results demonstrate that the ectopic expression of a gene encoding an enzyme involved in N assimilation or metabolism, for example, but not limited to AlaAT, results in increased plant biomass under a range of N conditions.

With reference to Figure 19B, it can be seen that control plants receiving additional N produced a higher yield of seed than plants grown under similar conditions but that did not receive any additional N. However, transgenic plants expressing btg-26/AlaAT exhibited higher yields than control plants in the presence or absence of additional N. Furthermore, the yields observed with the transgenic plants were equivalent on either high or low N. These results demonstrate that the ectopic expression of a gene encoding an enzyme involved in N assimilation or metabolism, for example, but not limited to AlaAT, results in increased seed yield, under a range ofN conditions.

Collectively these results indicate that transgenic plants ectopically expressing an enzyme involved in N assimilation or metabolism are capable of optimising the utilization of available N under a range of conditions of available N thereby resulting in an increase in plant biomass, seed yield or a combination thereof.

## Claims

1. A method for directing root-specific expression of a target gene, comprising: producing a plant from a transformed plant cell such that expression of a target gene occurs preferentially in the root, wherein the transformed plant cell comprises a target gene in operative linkage with a brassica turgor gene 26 promoter element as defined in SEQ ID NO: 1, or a fragment thereof which fragment directs expression of the target gene preferentially in the root.

2. The method of claim 1, wherein expression of the target gene is further environmentally or developmentally regulated.

3. The method of claim 1 or claim 2, wherein the target gene encodes an enzyme involved in nitrogen assimilation and/or metabolism.

4. The method of claim 3, wherein the enzyme is chosen from alanine dehydrogenase, glutamine synthetase, asparagine synthetase, glutamate synthase, asparaginase, alanine aminotransferase, aspartate aminotransferase and glutamate dehydrogenase.

5. The method of claim 4, wherein the enzyme is chosen from alanine aminotransferase and aspartate aminotransferase.

6. The method of claim 4, wherein the enzyme is alanine aminotransferase.

7. The method of any one of claims 1-6, wherein the plant is canola, barley, corn, rice, tobacco, soybean, cotton, alfalfa, tomato, wheat or potato.

8. The method of any one of claims 1-7, wherein the fragment of SEQ ID NO: I is the fragment defined in SEQ ID NO: 2.

9. The method of any one of claims 1-7, wherein the fragment of SEQ ID NO: 1 is the fragment defined in SEQ ID NO: 3.

10. The method of any one of claims 1-7, wherein the fragment of SEQ ID NO: I is the fragment defined in SEQ ID NO: 4.

11. The method of any one of claims 1-7, wherein the fragment of SEQ ID NO: 1 is the fragment defined in SEQ ID NO: 5.

12. The method of any one of claims 1-7, wherein the fragment of SEQ ID NO: 1 is the fragment defined in SEQ ID NO: 6.

## Patentansprüche

1. Ein Verfahren zum Lenken einer wurzel-spezifischen Expression eines Zielgens, umfassend:
Erzeugen einer Pflanze aus einer transformierten Pflanzenzelle derart, dass die Expression eines Zielgens vorzugsweise in der Wurzel stattfindet, wobei die transformierte Pflanzenzelle ein Zielgen in Wirkverbindung mit einem Brassica-Turgor-Gen-26-Promotor-Element, wie es in SEQ ID No. 1 definiert ist, oder einem Fragment desselben, welches die Expression des Zielgens vorzugsweise in die Wurzel lenkt, aufweist.

2. Das Verfahren nach Anspruch 1, wobei die Expression des Zielgens ferner umweltabhängig oder entwicklungsabhängig reguliert ist.

3. Das Verfahren nach Anspruch 1 oder Anspruch 2, wobei das Zielgen ein an der Stickstoffassimilation und/oder dem Stickstoffstoffwechsel beteiligtes Enzym kodiert.

4. Das Verfahren nach Anspruch 3, wobei das Enzym aus Alanindehydrogenase, Glutaminsynthetase, Asparaginsynthetase, Glutamatsynthase, Asparaginase, Alaninaminotransferase, Aspartataminotransferase und Glutamatdehydrogenase ausgewählt ist.

5. Das Verfahren nach Anspruch 4, wobei das Enzym ausgewählt ist aus Alaninaminotransferase und Aspartataminotransferase.

6. Das Verfahren nach Anspruch 4, wobei das Enzym Alaninaminotransferase ist.

7. Das Verfahren nach einem der Ansprüche 1 bis 6, wobei die Pflanze eine Raps-, Gerste-, Mais-, Reis-, Tabak-, Sojabohnen-, Baumwoll-, Luzerne-, Tomaten-, Weizen- oder Kartoffelpflanze ist.

8. Das Verfahren nach einem der Ansprüche 1 bis 7, wobei das Fragment von SEQ ID No. 1 das in SEQ ID No. 2 definierte Fragment ist.

9. Das Verfahren nach einem der Ansprüche 1 bis 7, wobei das Fragment von SEQ ID Nr. 1 das in SEQ ID Nr. 3 definierte Fragment ist.

10. Das Verfahren nach einem der Ansprüche 1 bis 7, wobei das Fragment von SEQ ID Nr. 1 das in SEQ ID Nr. 4 definierte Fragment ist.

11. Das Verfahren nach einem der Ansprüche 1 bis 7, wobei das Fragment von SEQ ID Nr. 1 das in SEQ ID Nr. 5 definierte Fragment ist.

12. Das Verfahren nach einem der Ansprüche 1 bis 7, wobei das Fragment von SEQ ID Nr. 1 das in SEQ ID Nr. 6 definierte Fragment ist.

## Revendications

1. Procédé pour diriger l'expression spécifique de racine d'un gène cible, comprenant : la production d'une plante à partir d'une cellule végétale transformée de sorte que l'expression d'un gène cible se produit de préférence dans la racine, dans lequel la cellule végétale transformée comprend un gène cible en liaison opérationnelle avec un promoteur du gène 26 de Brassica turgor tel que défini dans la SEQ ID NO:1 ou un fragment de celui-ci, lequel fragment dirige l'expression du gène cible de préférence dans la racine.

2. Procédé selon la revendication 1, dans lequel l'expression du gène cible est en outre régulée au plan environnemental ou du développement.

3. Procédé selon la revendication 1 ou la revendication 2, dans lequel le gène cible code pour une enzyme impliquée dans l'assimilation et/ou le métabolisme de l'azote.

4. Procédé selon la revendication 3, dans lequel l'enzyme est choisie parmi l'alanine déshydrogénase, la glutamine synthétase, l'asparagine synthétase, la glutamate synthétase, l'asparaginase, l'alanine aminotransférase, l'aspartate aminotransférase et la glutamate déshydrogénase.

5. Procédé selon la revendication 4, dans lequel l'enzyme est choisie parmi l'alanine aminotransférase et l'aspartate aminotransférase.

6. Procédé selon la revendication 4, dans lequel l'enzyme est l'alanine aminotransférase.

7. Procédé selon l'une quelconque des revendications 1 à 6, dans lequel la plante est le colza, l'orge, le maïs, le riz, le tabac, le soja, le coton, la luzerne, la tomate, le blé ou la pomme de terre.

8. Procédé selon l'une quelconque des revendications 1 à 7, dans lequel le fragment de la SEQ ID NO:1 est le fragment défini dans la SEQ ID NO:2.

9. Procédé selon l'une quelconque des revendications 1 à 7, dans lequel le fragment de la SEQ ID NO:1 est le fragment défini dans la SEQ ID NO:3.

10. Procédé selon l'une quelconque des revendications 1 à 7, dans lequel le fragment de la SEQ ID NO:1 est le fragment défini dans la SEQ ID NO:4.

11. Procédé selon l'une quelconque des revendications 1 à 7, dans lequel le fragment de la SEQ ID NO:1 est le fragment défini dans la SEQ ID NO:5.

12. Procédé selon l'une quelconque des revendications 1 à 7, dans lequel le fragment de la SEQ ID NO:1 est le fragment défini dans la SEQ ID NO:6.


### SEQUENCE LISTING

<110> The Govenors of the University of Alberta
   Good, Allen
<120> Tissue-Specific Expression of Target Genes in Plants
   <130> 08-890287WO
<140> PCT/CA01/00066
   <141> 2001-01-02
<150> US 09/493,803
   <151> 2000-01-28
<160> 8
   <170> Patent In version 3.0
<210> 1
   <211> 365
   <212> DNA
   <213> Brassica napus
   <220>
   <221> misc_feature
   <223> btg-26 promoter
<400> 1
   gtcgacctgc aggtcaacgg atcctaatcg gggtatatcc cgacccggaa aaagaaacgt     60
   aggacacgtg acaaaacttc atatgatccg agtgaatcaa gccaaaaggg ggattgacac    120
   aacagctcag ctttcgtttt cggtccaatc gctgttccaa ctttacttac aagtcgtaca    180
   cgtctctctc tctctctctc tctctcactc acttcctctt ataaagactc tctgatcaaa    240
   cgtataatcg gaaaactcca ttctttgata ccatcgataa tactaagaga ggtgattgat    300
   tctttaatca ctgtttgata tccttaactt tgatccattt actctgttca atcatttttg    360
   tagag                                                                365
<210> 2
   <211> 6
   <212> DNA
   <213> Brassica napus
   <220>
   <221> misc_feature
   <223> fragment of btg-26 promoter
<400> 2
   caacgg 6
<210> 3
   <211> 11
   <212> DNA
   <213> Brassica napus
   <220>
   <221> misc_feature
   <223> fragment of btg-26 promoter
<400> 3
   ggacacgtga c 11
<210> 4
   <211> 6
   <212> DNA
   <213> Brassica napus
<400> 4
   caacag 6
<210> 5
   <211> 6
   <212> DNA
   <213> Brassica napus
   <220>
   <221> misc_feature
   <223> fragment of btg-26 promoter
<400> 5
   caactt 6
<210> 6
   <211> 38
   <212> DNA
   <213> Brassica napus
   <220>
   <221> misc_feature
   <223> fragment of btg-26 promoter
<400> 6
   tctctctctc tctctctctc tctcactcac ttcctctt 38
<210> 7
   <211> 1701
   <212> DNA
   <213> Hordeum vulgare
   <220>
   <221> CDS
   <222> (95)..(1540)
<400> 7
   ggccacaaaa ccgcggaaag agatagacgg acagctagag gcgtcggaag atactcgctg      60
   ctctgccgcc cccttcgtct tagttgatct cgcc atg gct gcc acc gtc gcc gtg     115
                                         Met Ala Ala Thr Val Ala Val
                                         1               5
   gac aac ctg aac ccc aag gtt tta aaa tgt gag tat gct gtg cgt gga       163
   Asp Asn Leu Asn Pro Lys Val Leu Lys Cys Glu Tyr Ala Val Arg Gly
           10                  15                  20
   gag att gtc atc cat gct cag cgc ttg cag gaa cag cta aag act caa       211
   Glu Ile Val Ile His Ala Gln Arg Leu Gln Glu Gln Leu Lys Thr Gln
       25                  30                  35
   cca ggg tct cta cct ttt gat gag atc ctc tat tgt aac att ggg aac       259
   Pro Gly Ser Leu Pro Phe Asp Glu Ile Leu Tyr Cys Asn Ile Gly Asn
   40                  45                  50                  55
   cca caa tct ctt ggt cag caa cca gtt aca ttc ttc agg gag gtt ctt       307
   Pro Gln Ser Leu Gly Gln Gln Pro Val Thr Phe Phe Arg Glu Val Leu
                   60                  65                  70
   gcc ctt tgt gat cat cca gac ctg ttg caa aga gag gaa atc aaa aca      355
   Ala Leu Cys Asp His Pro Asp Leu Leu Gln Arg Glu Glu Ile Lys Thr
               75                  80                  85
   ttg ttc agt gct gat tct att tct cga gca aag cag att ctt gcc atg      403
   Leu Phe Ser Ala Asp Ser Ile Ser Arg Ala Lys Gln Ile Leu Ala Met
           90                  95                  100
   ata cct gga aga gca aca gga gca tac agc cat agc cag ggt att aaa      451
   Ile Pro Gly Arg Ala Thr Gly Ala Tyr Ser His Ser Gln Gly Ile Lys
       105                 110                 115
   gga ctt cgt gat gca att gct tct ggg atc gct tca cga gat gga ttc      499
   Gly Leu Arg Asp Ala Ile Ala Ser Gly Ile Ala Ser Arg Asp Gly Phe
   120                 125                 130                 135
   cct gct aat gct gat gac att ttt ctc aca gat gga gca agt cct ggg      547
   Pro Ala Asn Ala Asp Asp Ile Phe Leu Thr Asp Gly Ala Ser Pro Gly
                   140                 145                 150
   gtg cac ctg atg atg caa tta ctg ata agg aat gag aaa gat ggc att      595
   Val His Leu Met Met Gln Leu Leu Ile Arg Asn Glu Lys Asp Gly Ile
               155                 160                 165
   ctt gtc ccg att cct cag tac ccc ttg tac tcg gct tcc ata gct ctt      643
   Leu Val Pro Ile Pro Gln Tyr Pro Leu Tyr Ser Ala Ser Ile Ala Leu
           170                 175                 180
   cat ggc gga gct ctt gtc cca tac tat ctc aat gaa tcg acg ggc tgg      691
   His Gly Gly Ala Leu Val Pro Tyr Tyr Leu Asn Glu Ser Thr Gly Trp
       185                 190                 195
   ggt ttg gaa acc tct gat gtt aag aag caa ctt gaa gat gct cgg tca      739
   Gly Leu Glu Thr Ser Asp Val Lys Lys Gln Leu Glu Asp Ala Arg Ser
   200                 205                 210                 215
   aga ggc atc aac gtt agg gct ttg gtg gtt atc aat cca gga aat cca      787
   Arg Gly Ile Asn Val Arg Ala Leu Val Val Ile Asn Pro Gly Asn Pro
                   220                 225                 230
   act gga cag gta ctt gct gaa gaa aac caa tat gac ata gtg aag ttc      835
   Thr Gly Gln Val Leu Ala Glu Glu Asn Gln Tyr Asp Ile Val Lys Phe
               235                 240                 245
   tgc aaa aat gag ggt ctt gtt ctt cta gct gat gag gta tac caa gag      883
   Cys Lys Asn Glu Gly Leu Val Leu Leu Ala Asp Glu Val Tyr Gln Glu
           250                 255                 260
   aac atc tat gtt gac aac aag aaa ttc cac tct ttc aag aag ata gtg      931
   Asn Ile Tyr Val Asp Asn Lys Lys Phe His Ser Phe Lys Lys Ile Val
       265                 270                 275
   aga tcc ttg gga tac ggc gag gag gat ctc cct cta gta tca tat caa      979
   Arg Ser Leu Gly Tyr Gly Glu Glu Asp Leu Pro Leu Val Ser Tyr Gln
   280                 285                 290                 295
   tct gtt tct aag gga tat tat ggt gag tgt ggt aaa aga ggt ggt tac     1027
   Ser Val Ser Lys Gly Tyr Tyr Gly Glu Cys Gly Lys Arg Gly Gly Tyr
                   300                 305                 310
   ttt gag att act ggc ttc agt gct cca gta aga gag cag atc tac aaa      1075
   Phe Glu Ile Thr Gly Phe Ser Ala Pro Val Arg Glu Gln Ile Tyr Lys
               315                 320                 325
   ata gca tca gtg aac cta tgc tcc aat atc act ggc cag atc ctt gct      1123
   Ile Ala Ser Val Asn Leu Cys Ser Asn Ile Thr Gly Gln Ile Leu Ala
           330                 335                 340
   agt ctt gtc atg aac cca cca aag gct agt gat gaa tca tac gct tca      1171
   Ser Leu Val Met Asn Pro Pro Lys Ala Ser Asp Glu Ser Tyr Ala Ser
       345                 350                 355
   tac aag gca gaa aaa gat gga atc ctc gca tct tta gct cgt cgt gcg      1219
   Tyr Lys Ala Glu Lys Asp Gly Ile Leu Ala Ser Leu Ala Arg Arg Ala
   360                 365                 370                 375
   aag gca ttg gag cat gca ttc aat aaa ctt gag gga att act tgc aac      1267
   Lys Ala Leu Glu His Ala Phe Asn Lys Leu Glu Gly Ile Thr Cys Asn
                   380                 385                 390
   gag gct gaa gga gca atg tac gtg ttc cct caa atc tgt ctg cca cag      1315
   Glu Ala Glu Gly Ala Met Tyr Val Phe Pro Gln Ile Cys Leu Pro Gln
               395                 400                 405
   aag gca att gag gct gct aaa gct gct aac aaa gca cct gat gca ttc      1363
   Lys Ala Ile Glu Ala Ala Lys Ala Ala Asn Lys Ala Pro Asp Ala Phe
           410                 415                 420
   tat gct ctt cgt ctc ctc gag tcg act gga atc gtc gtt gtc cct gga      1411
   Tyr Ala Leu Arg Leu Leu Glu Ser Thr Gly Ile Val Val Val Pro Gly
       425                 430                 435
   tca gga ttt ggc cag gtt cct ggc aca tgg cac ttc agg tgc acg atc      1459
   Ser Gly Phe Gly Gln Val Pro Gly Thr Trp His Phe Arg Cys Thr Ile
   440                 445                 450                 455
   ctt ccg cag gag gat aag atc ccg gca gtc atc tcc cgc ttc acg gtg      1507
   Leu Pro Gln Glu Asp Lys Ile Pro Ala Val Ile Ser Arg Phe Thr Val
                   460                 465                 470
   ttc cat gag gcg ttc atg tca gag tat cgt gac taaactggtg caacatgtgg    1560
   Phe His Glu Ala Phe Met Ser Glu Tyr Arg Asp
               475                 480
   gattacatac aaccctcatg gggttttcgt aggcgttctt ggttttgccc ccccccccct    1620
   tctctctctc tctctctctg acagcatcct cctctagatg agacaaaata aagcaaagcc    1680
   atgtcatcct taaaaaaaaa a                                              1701
<210> 8
   <211> 482
   <212> PRT
   <213> Hordeum vulgare
<400> 8
   Met Ala Ala Thr Val Ala Val Asp Asn Leu Asn Pro Lys Val Leu Lys
   1               5                   10                  15
   Cys Glu Tyr Ala Val Arg Gly Glu Ile Val Ile His Ala Gln Arg Leu
               20                  25                  30
   Gln Glu Gln Leu Lys Thr Gln Pro Gly Ser Leu Pro Phe Asp Glu Ile
           35                  40                  45
   Leu Tyr Cys Asn Ile Gly Asn Pro Gln Ser Leu Gly Gln Gln Pro Val
       50                  55                  60
   Thr Phe Phe Arg Glu Val Leu Ala Leu Cys Asp His Pro Asp Leu Leu
   65                  70                  75                  80
   Gln Arg Glu Glu Ile Lys Thr Leu Phe Ser Ala Asp Ser Ile Ser Arg
                   85                  90                  95
   Ala Lys Gln Ile Leu Ala Met Ile Pro Gly Arg Ala Thr Gly Ala Tyr
               100                 105                 110
   Ser His Ser Gln Gly Ile Lys Gly Leu Arg Asp Ala Ile Ala Ser Gly
           115                 120                 125
   Ile Ala Ser Arg Asp Gly Phe Pro Ala Asn Ala Asp Asp Ile Phe Leu
       130                 135                 140
   Thr Asp Gly Ala Ser Pro Gly Val His Leu Met Met Gln Leu Leu Ile
   145                 150                 155                 160
   Arg Asn Glu Lys Asp Gly Ile Leu Val Pro Ile Pro Gln Tyr Pro Leu
                   165                 170                 175
   Tyr Ser Ala Ser Ile Ala Leu His Gly Gly Ala Leu Val Pro Tyr Tyr
               180                 185                 190
   Leu Asn Glu Ser Thr Gly Trp Gly Leu Glu Thr Ser Asp Val Lys Lys
           195                 200                 205
   Gln Leu Glu Asp Ala Arg Ser Arg Gly Ile Asn Val Arg Ala Leu Val
       210                 215                 220
   Val Ile Asn Pro Gly Asn Pro Thr Gly Gln Val Leu Ala Glu Glu Asn
   225                 230                 235                 240
   Gln Tyr Asp Ile Val Lys Phe Cys Lys Asn Glu Gly Leu Val Leu Leu
                   245                 250                 255
   Ala Asp Glu Val Tyr Gln Glu Asn Ile Tyr Val Asp Asn Lys Lys Phe
               260                 265                 270
   His Ser Phe Lys Lys Ile Val Arg Ser Leu Gly Tyr Gly Glu Glu Asp
           275                 280                 285
   Leu Pro Leu Val Ser Tyr Gln Ser Val Ser Lys Gly Tyr Tyr Gly Glu
       290                 295                 300
   Cys Gly Lys Arg Gly Gly Tyr Phe Glu Ile Thr Gly Phe Ser Ala Pro
   305                 310                 315                 320
   Val Arg Glu Gln Ile Tyr Lys Ile Ala Ser Val Asn Leu Cys Ser Asn
                   325                 330                 335
   Ile Thr Gly Gln Ile Leu Ala Ser Leu Val Met Asn Pro Pro Lys Ala
               340                 345                 350
   Ser Asp Glu Ser Tyr Ala Ser Tyr Lys Ala Glu Lys Asp Gly Ile Leu
           355                 360                 365
   Ala Ser Leu Ala Arg Arg Ala Lys Ala Leu Glu His Ala Phe Asn Lys
       370                 375                 380
   Leu Glu Gly Ile Thr Cys Asn Glu Ala Glu Gly Ala Met Tyr Val Phe
   385                 390                 395                 400
   Pro Gln Ile Cys Leu Pro Gln Lys Ala Ile Glu Ala Ala Lys Ala Ala
                   405                 410                 415
   Asn Lys Ala Pro Asp Ala Phe Tyr Ala Leu Arg Leu Leu Glu Ser Thr
               420                 425                 430
   Gly Ile Val Val Val Pro Gly Ser Gly Phe Gly Gln Val Pro Gly Thr
           435                 440                 445
   Trp His Phe Arg Cys Thr Ile Leu Pro Gln Glu Asp Lys Ile Pro Ala
       450                 455                 460
   Val Ile Ser Arg Phe Thr Val Phe His Glu Ala Phe Met Ser Glu Tyr
   465                 470                 475                 480
   Arg Asp